(19) Europäisches Patentamt
European Patent Office
Office européen des brevets

(11) Veröffentlichungsnummer: **0 436 936 A2**

# (12) EUROPÄISCHE PATENTANMELDUNG

(21) Anmeldenummer: **90125641.2**

(22) Anmeldetag: **28.12.90**

(51) Int. Cl.[5]: **C07J 41/00**, C07J 9/00, C07J 33/00, C07J 31/00, C07J 17/00, C07F 9/655, C07F 9/6558, C07D 311/66, C07D 339/00, C07D 319/06, C07C 69/88

(30) Priorität: **09.01.90 DE 4000397**

(43) Veröffentlichungstag der Anmeldung:
**17.07.91 Patentblatt 91/29**

(84) Benannte Vertragsstaaten:
**AT BE CH DE DK ES FR GB GR IT LI LU NL SE**

(71) Anmelder: **HOECHST AKTIENGESELLSCHAFT**
**Postfach 80 03 20**
**W-6230 Frankfurt am Main 80(DE)**

(72) Erfinder: **Weithmann, Klaus-Ulrich, Dr.**
**Am Domherrenwald 18**
**W-6238 Hofheim am Taunus(DE)**
Erfinder: **Wess, Günther, Dr.**
**Langenselbolder Weg 35**
**W-6455 Erlensee(DE)**
Erfinder: **Seiffge, Dirk, Dr.**
**Kostheimer Landstrasse 11**
**W-6502 Mainz(DE)**

## (54) Lipidselektive Antioxidantien sowie deren Herstellung und Verwendung.

(57) Lipidselektive Antioxidantien der allgemeinen Formel I

$(A)_a(L)(X)_{a'}$ (I),

worin
A = antioxidative Komponente,
L = Brückenglied,
X = lipophile Komponente
a und a' = unabhängig voneinander die Zahlen 1 oder 2.

Die Verbindungen werden verwendet zum Schutz von lipidhaltigen Substanzen gegen Oxidation sowie in Arzneimitteln zur Prophylaxe und Therapie von Krankheiten, bei denen Bioradikale involviert sind, insbesondere von Herz-Kreislauf- und Gefäßkrankheiten.

EP 0 436 936 A2

# LIPIDSELEKTIVE ANTIOXIDANTIEN SOWIE DEREN HERSTELLUNG UND VERWENDUNG

Antioxidantien werden in der Lebensmittelindustrie als Konservierungszusätze verwendet; denn Nahrungsmittel können beim Aufbewahren unerwünschte oxidative Veränderungen erleiden. Es ist auch bekannt, daß die Lipidbestandteile der Lebensmittel besonders oxidationsempfindlich sind und bei Lagerung an der Luft ranzig werden, weil sich chemisch über teilweise radikalische Zwischenstufen Peroxide und ungesättigte Aldehyde bilden. Ähnliche unerwünschte Prozesse spielen sich auch bei der Alterung von Stoffen ab, die aus längeren Kohlenstoffketten bestehen, z.B. Kautschuk, Plastik und Mineralöl. Bekanntlich werden z.B. das lipidlösliche BHA (butyliertes Hydroxyanisol, vgl. Merck-Index, tenth edition, Rahway, USA, 1983 Nr. 1521 Seite 215), das noch besser lipidlösliche BHT (butyliertes Hydroxytoluol, ibid, Nr. 1520), und das ebenfalls lipidlösliche, aber unstabile, temperatur- und lichtempfindliche Vitamin E (ibid, Nr. 9832, Seite 1437) sowie die lipidunlösliche Ascorbinsäure (ibid, Nr. 846 Seite 120) als Konservierungsmittel verwendet.

Gegenstand der vorliegenden Erfindung sind nun neue Antioxidantien mit besonders vorteilhaften Wirkungen in lipophilem Milieu. Es handelt sich um Verbindungen der allgemeinen Formel I

$(A)_a(L)(X)_{a'}$ (I),

worin
a, a', A, L und X die folgende Bedeutung besitzen:
a und a' = unabhängig voneinander die Zahlen 1 oder 2,
A = antioxidative Komponente aus der Gruppe
$A_1$ - Chromanteilstruktur des Vitamins E

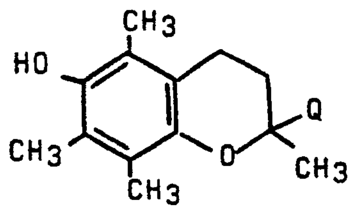

worin Q in dieser und allen folgenden Formeln eine freie Valenz (kovalente Einfachbindung) darstellt,
$A_2$ - alkylsubstituierter Mono-, Di- oder Tri-Phenol-Rest

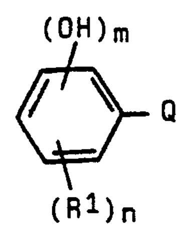

worin
m = 1 oder 2,
n = 1 oder 2, und
m + n = 3 oder 4,
$R^1$ = Alkylrest und/oder Alkoxyrest
und die Gesamtzahl der C-Atome des Alkyl- bzw. Alkoxyrestes bzw. der Alkyl- und Alkoxyreste = maximal 8 ist;
$A_3$ - Reductonrest

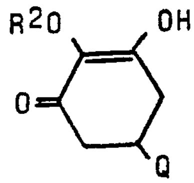

oder

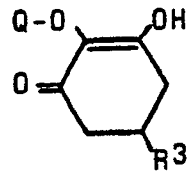

worin

$R^2$ = H oder niederer Alkylrest (vorzugsweise $C_1$-$C_4$) und

$R^3$ = H, $COOR^4$, $CH_2OR^4$

$R^4$ = H oder niederer Alkylrest (vorzugsweise $C_1$-$C_4$)

$A_4$ - 1,2-Dithiacycloalkyl oder 1,2-Dithiacycloalkenyl-Rest mit 2 - 6, vorzugsweise 2 - 4 C-Atomen im Ring und die durch Hydrogenierung reduzierte Dithiolform dieser Reste

$A_5$ - Ascorbinsäure(-Derivat)-Rest

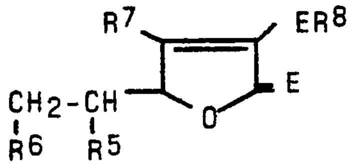

worin

E = O, S oder $NR^9$

$R^5$ = H, EH, EQ oder Q

$R^6$ = H, EH, EQ-(L-$X_1$) oder Q-(L-$X_1$)

$R^7$ = H, EH, EQ, Q oder einer der unter $A_2$ und $A_3$ genannten Reste,

$R^8$ = H, EH, Q-(L-$X_1$) oder -PO($OR^9$)$_2$,

$R^9$ = H, niederer Alkylrest (vorzugsweise $C_1$-$C_4$) oder Q,

und nur 1 oder 2 - bevorzugt 1 - der Reste $R^5$ -$R^9$ gleich Q sind bzw. Q enthalten,

L = Brückenglied und

$X_1$ = lipophile Komponente wie nachstehend definiert;

L = Brückenglied,

bestehend aus einem oder mehreren der Bausteine

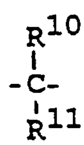

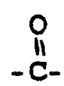

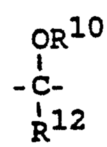

-O- , -S- , -$NR^{10}$- und 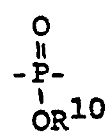

worin

$R^{10}$, $R^{11}$, $R^{12}$ = H, niederer Alkylrest (vorzugsweise $C_1$-$C_4$) oder Q,

$R^{11}$ darüber hinaus auch noch -$CO_aR^{10}$ sein kann (mit a = 1 oder 2),

und 2 Reste der Art -O-, -S- und/oder -$NR^{10}$- durch mindestens 1 C- oder P-Atom voneinander getrennt sind;

X = lipophile Komponente aus der Gruppe

$X_1$ - Cholanderivat-Reste

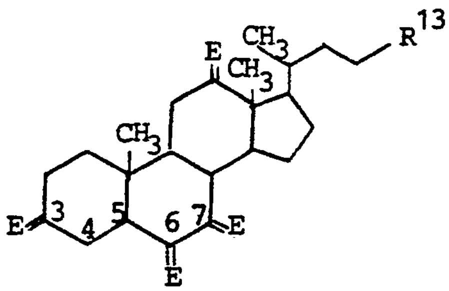

worin
$R^{13}$ = sec. $C_4H_9$ (= Cholestan), $R^{11}$ (s. bei L) oder Q,
E = O, S, $NR^{10}$ ($R^{10}$ s. bei L), (α,β-OH,H) oder (α,β-Q, H) und in 4,5- bzw. 5,6- bzw. 7,8-Position eine Doppelbindung vorhanden sein kann, und
$X_2$ - Alkyl- oder Cycloalkylrest oder Fettsäurederivat-Rest mit bis zu 24 C-Atomen.

Unter den Komponenten A, L und X sind folgende Reste bevorzugt:
für $A_4$:
ein Rest der folgenden Formeln in der Dithiaform (gemäß den Formeln) oder in der durch Hydrogenierung reduzierten Dithiolform:

$A_{4.1}$

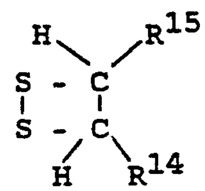

$A_{4.2}$

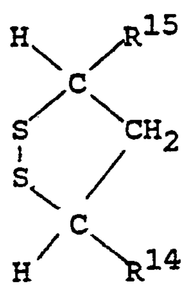

worin
$R^{14}$ = H oder niederer Alkylrest (vorzugsweise $C_1$-$C_4$), und
$R^{15}$ = $-(CH_2)_b-Q$
b = 0 - 12, vorzugsweise 0 - 4.

Im Fall $A_{4.2}$ ist besonders bevorzugt:
$R^{14}$ = H und
$R^{15}$ = $-(CH_2)_4-Q$
(= Decarboxy-Liponsäure- bzw. -Dihydroliponsäure-Teilstruktur).

$A_{4.3}$

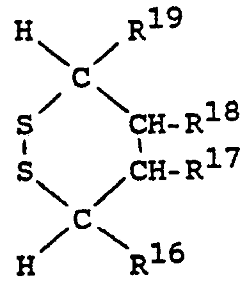

worin

| | |
|---|---|
| $R^{16}$ und $R^{19}$ = | unabhängig voneinander = H oder niederer Alkylrest (vorzugsweise $C_1$-$C_4$) |
| $R^{17}$ = | Q und |
| $R^{18}$ = | H, niederer Alkylrest (vorzugsweise $C_1$-$C_4$),Acylrest $OCOR^{19}$ oder $OR^{19}$ |
| $R^{19}$ = | niederer Alkylrest (vorzugsweise $C_1$-$C_4$) oder Q. |

$A_{4.4}$ **Dithiothreit- oder Dithioerythrit-Teilstruktur**

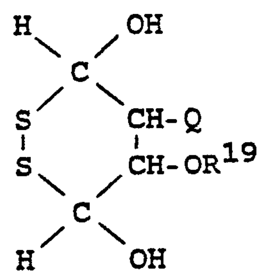

worin
$R^{19}$ die gleiche Bedeutung wie bei 4.3 besitzt.

$A_{4.5}$

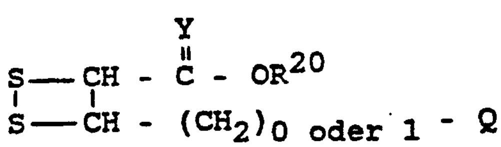

worin

| | |
|---|---|
| $R^{20}$ = | H oder niederer Alkylrest (vorzugsweise $C_1$-$C_4$) und |
| Y = | $H_2$ oder O. |

für $A_5$:
E = O
$R^5$, $R^6$ und $R^7$ = unabhängig voneinander = OH oder OQ,
$R^8$ = H oder Q,
wobei nur 1 oder 2 Reste $R^5$ - $R^8$ Q enthalten bzw. gleich Q sind (= Ascorbinsäurerest).

Weitere besonders bevorzugte Reste A sind:

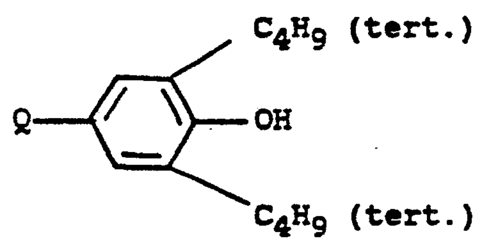

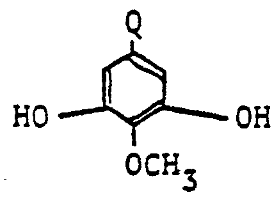

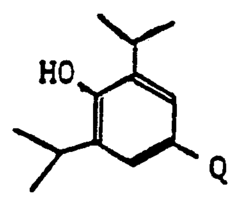

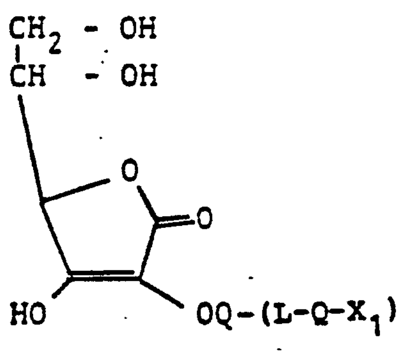

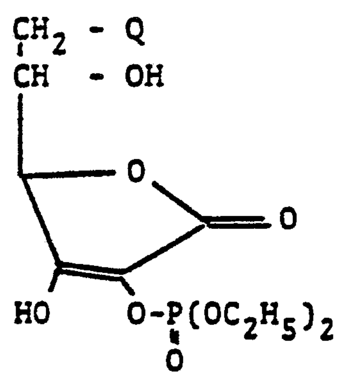

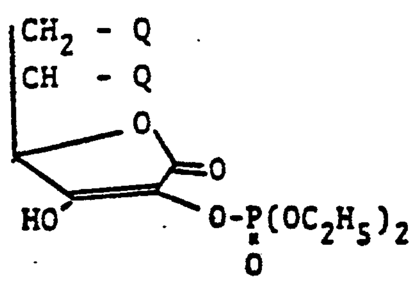

L ist ein hinsichtlich der antioxidativen Wirkung inertes, chemisch stabiles Brückenglied zur Verknüpfung von A und X. Brückenglieder L, welche eine Esterbindung enthalten, sind etwas hydrolyseempfindlicher als Brückenglieder ohne Esterkomponente. Dies muß bedacht werden, wenn die stabilisierenden Lipide mit Säuren oder Laugen in Berührung kommen. Z.B. bei der Verwendung als Arzneimittel kann die Spaltung der Esterbindung durch Enzyme am pharmakologischen Wirkort aber auch Vorteile bieten, indem die antioxidative Komponente genau am Wirkort abgespalten und aufkonzentriert wird.

L besitzt vorzugsweise die folgende allgemeine Formel:

$$L = M_p\{[-(CH_2)_w-(G_1)_x-(G_2)]_v-(CH_2)_y-(G_3)_z-(G_4)_{p+1}\}M_p$$

worin
p, x und z unabhängig voneinander = 0 oder 1,
v, w und y unabhängig voneinander = 0 - 4, und
v+w+y+z = 0 - 10,

$M = -CR^{10}=$ ,

$-N=$ oder

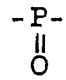

$G_1$, $G_2$, $G_3$ und $G_4$ unabhängig voneinander = -O- , -S- , $-NR^{10}-$ ,

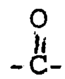 , $-CHOR^{10}-$ oder $-CH(CH_2-OR^{10})-$ ,

wobei $R^{10}$ die vorher genannte Bedeutung besitzt (= H, niederer Alkylrest oder Q) und 2 der Reste -O-, -S-, und/oder $-NR^{10}-$ durch mindestens 1 C-Atom voneinander getrennt sind.

Besonders bevorzugt ist L ein Rest aus der Gruppe:

$L_1$ : $Q-O-(CH_2)_r-O-CO-Q$
$L_2$ : $Q-CO-NH-(CH_2)_q-NH-CO-Q$
$L_3$ : $Q-O-(CH_2-)_r-NH-CO-Q$
$L_4$ : $Q-(CH_2-)_r(-O-)_b-Q$
$L_5$ : $Q-(CH_2-)_s-O-(CH_2-)_r-O-Q$
$L_6$ : $Q-(CH_2-)_s-NH-(CH_2-)_r-O-Q$
$L_7$ : $Q-CO-NH-(CH_2-CH_2)_r-O-Q$
$L_8$ : $Q-O-(CH_2-)_s-CHOH-(CH_2-)_s-O-(CH_2-)_s-Q$

$L_9$ : 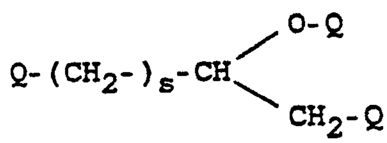

$L_{10}$: $Q-(CH_2-)_q-Q$
$L_{11}$: $Q-(CH_2-)_s-CHCO_2R^{10}-CHOH-Q$
$L_{12}$: $Q-CH=C(CO_2R^{10})-CO-Q$
$L_{13}$: $Q-CO-NH-(CH_2-)q-NH-CO-Q$
$L_{14}$: $Q-(CH_2-)_s-O-(CH_2-)_r-O-$

$L_{15}$: 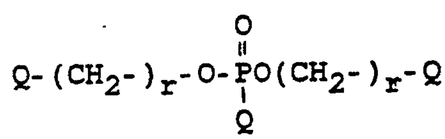

$L_{16}$: $[Q-(CH_2-)_2-O-(CH_2)_s]_2CH-Q$
$L_{17}$: $Q-O-(CH_2-)_s-CHOH-O-(CH_2-)_s-Q$
$L_{18}$: $Q-O-(CH_2-)_s-CH(CH_2-OH)-O-CO-Q$
$L_{19}$: $Q-O-(CH_2-)_s-CHOH-(CH_2-)_s-O-CO-O$
$L_{20}$: $Q-CO-NR^{10}-Q$
$L_{21}$: $Q-CO(O)_x-Q$
$L_{22}$: $Q-CH_2-N[CH(CH_3)_2]-(CH_2)_r-CHOHCH_2CHOHCH_2-CO(O)_x-Q$
$L_{23}$: $Q-(CH_2)_s-Q$
$L_{24}$: $Q-NR^{10}-Q$
$L_{25}$: $Q-O-Q$
$L_{26}$: $Q-(CH_2)_s-CHCO_2R^{10}-COQ$

$L_{27}$: 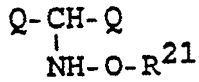

worin

$R^{21}$ = Benzyl- oder $R^{10}$

$L_{28}$: 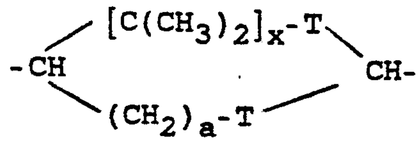

worin

T = O oder S
x = 0, 1
a = 1, 2;
q = 1 - 5, bevorzugt 3
r = 1 - 5, bevorzugt 2
s = 1 - 5, bevorzugt 1 bedeuten,
und $R^{10}$ die oben genannte Bedeutung besitzt
(= H, niederer Alkylrest oder Q).

Ganz besonders bevorzugte Brückenglieder L sind:
Q-CO-O-Q
Q-CO-NH-Q
Q-CO-NH-$CH_2$-$CH_2$-O-Q

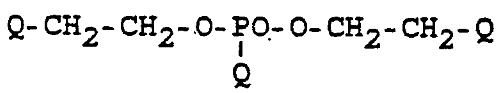

Q-CO-NH-$(CH_2)_3$-NH-CO-Q

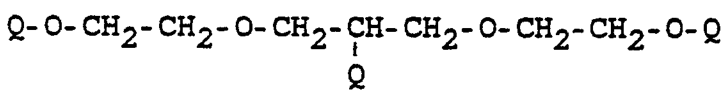

Q-CO-Q
Q-CO-NH-$(CH_2)_3$-NH-CO-Q
Q-$CH_2$-O-$CH_2$-$CH_2$-O-Q

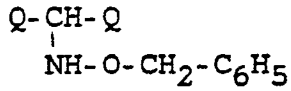

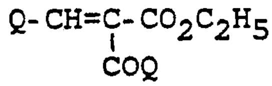

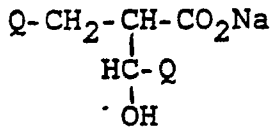

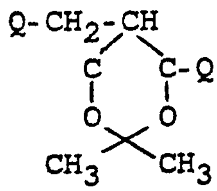

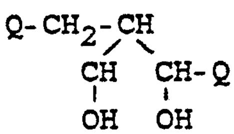

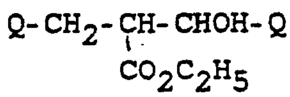

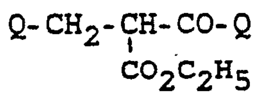

$Q-COO-CH_2-CHOH-CH_2-O-Q$

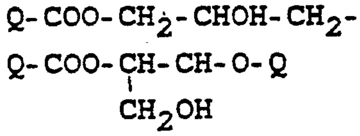

$Q-CH_2-O-CH_2-CHOH-CH_2-O-Q$

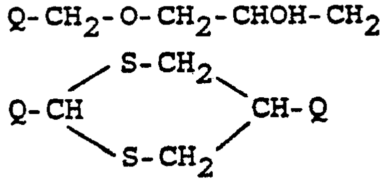

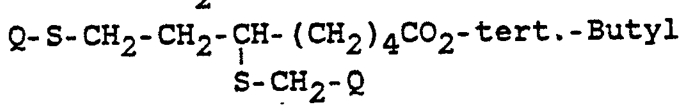

X ist bevorzugt ein Rest aus der folgenden Gruppe:

$X_{1.1}$ Cholesterol
$X_{1.2}$ Cholestanol
$X_{1.3}$ Cholsäure
$X_{1.4}$ Desoxycholsäure
$X_{1.5}$ Ursodesoxycholsäure
$X_{1.6}$ Chenodesoxycholsäure
sowie
$X_{2.1}$ $CH_3-(CH_2)_t-Q$
$X_{2.2}$ $Q-C(CH_3)_3$
$X_{2.3}$ $Q-CH(CH_2)d$
$X_{2.4}$ $Q-C\equiv C-(CH_2)_5-CH_3$
$X_{2.5}$ $R^{10}-CO_2-(CH_2)_z-Q$
d = 4 - 6

t = 3 - 24, vorzugsweise 6 - 18.
z = 0 oder 1
Besonders bevorzugte Reste $X_1$ sind:

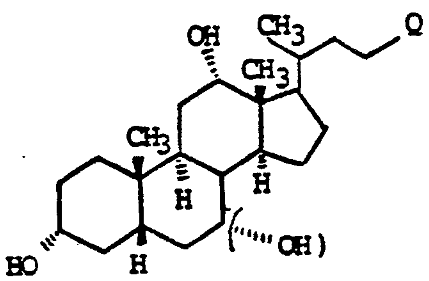

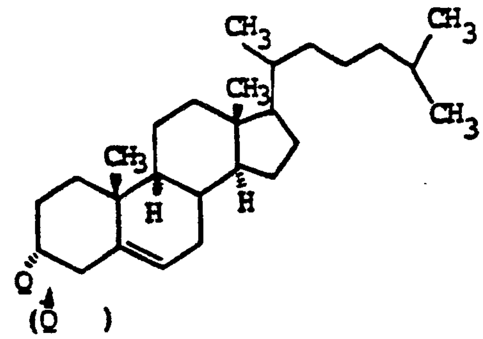

besonders bevorzugte Reste $X_2$:
$CH_3$-$(CH_2)_{16}$-Q
$CH_3$-$(CH_2)_{17}$-Q
$CH_3$-$(CH_2)_{18}$-Q
$CH_3$-$C(CH_2)_2$-Q
$CH_3$-$(CH_2)_5$-C≡C-Q.

**Herstellung der Verbindungen der Formel I:**

Die Herstellung der Verbindungen erfolgt nach Verfahren, die allgemein bekannt sind. Die Einzelkomponenten A und X werden frei oder geschützt eingesetzt, gegebenenfalls in Form reaktiver Derivate. Die Verknüpfung mit L erfolgt über ein reaktives Derivat von L. Im Falle der geschützten Verbindungen werden die Schutzgruppen im Anschluß an die Verknüpfung wieder abgespalten.

Das Verfahren stellt sich konkreter wie im experimentellen Teil beschrieben dar.

Die erfindungsgemäßen Verbindungen der Formel I können als Antioxidantien z.B. in der Fett, Öl, Plastik und Kautschuk verarbeitenden Industrie (wie der Lebensmittel-, Kosmetik-, Pharma-, Gummi- und Mineralöl-Industrie), als Konservierungsmittel für Fettstoffe (Lipide) bzw. für polymere langkettige Kohlenstoffverbindungen verwendet werden.

Wie nachstehend ausgeführt, hat auch die in vivo Oxidation von Lipidbestandteilen (z.B. von Blutfetten oder von Lipiden der Biomembranen) des menschlichen oder tierischen Körpers unerwünschte Folgen: Im Blut werden wichtige Lipide, insbesondere das Cholesterol, mit Hilfe des Low Density Lipoprotein (LDL) transportiert. Unter physiologischen Bedingungen steht das LDL mit dem Blutgefäßsystem in kontrollierter Wechselwirkung. Es wird über spezifische Rezeptoren in einem regulierten Prozeß in die Gefäßwand aufgenommen und stellt dort seine Lipidanteile als Energieträger oder als Zellbausteine zur Verfügung. Wenn nun keine ausreichende antioxidative Schutzwirkung vorhanden ist, z.B. insbesondere unter hyperlipidämischen Bedingungen, kann es zur Oxidation der Blutfette kommen. Die oxidierten Blutfette, bzw. LDL, werden dann unter Umgehung der spezifischen LDL-Rezeptoren ungehindert von den Gefäßwänden aufgenommen, d.h. der kontrollierte Prozeß der Rezeptor-Regulation entgleist. Im Verlauf dieser toxischen Prozesse, an denen insbesondere radikalische Zwischenstufen beteiligt sind, entstehen z.B. Oxidationsprodukte des Cholesterols mit mutagenen und zelltoxischen Eigenschaften (Proc. Natl. Acad. Sci. USA 81 (1984) 4198-4202), während die ungesättigten Fettsäurereste bis zu z.B. Hydroxyalkenalen mit starken biociden Wirkungen oxidativ abgebaut werden. Im weiteren Verlauf der Krankheit werden die befallenen Gefäßbezirke durch die sogenannte Schaumzellbildung unter Beteiligung von Makrophagen erheblich geschädigt. Es kommt zur Proliferation der glatten Gefäßmuskulatur und schließlich zur Ausbildung von atherosklerotischen Plaques, die die Blutbahn verengen. Dort können sich Blutgerinnsel festsetzen und schließlich kann ein Infarkt zu bleibenden Schäden oder zum Tode des Patienten führen. Diese pathologischen Prozesse können allein durch diätetische Maßnahmen zur Reduktion des Blut-Lipidspiegels nicht vollständig verhindert werden. Die medikamentöse Senkung der Blut-Lipidspiegel ist zwar Stand der Technik, hat aber den Nachteil, daß sie in die komplexen Lipid-Stoffwechselvorgänge eingreift. Unter physiologischen Bedingungen stehen diese Stoffwechselvorgänge in einem genau ausgewogenen Gleichgewicht. Eine Beeinflussung dieses Gleichgewichtes, insbesondere über einen längeren Zeitraum hinweg, wird zwangsläufig auch zu unerwünschten biologischen Reaktionen führen. Unerwünschte Nebenwirkungen von

lipidsenkenden Medikamenten, wie Clofibrat oder Nikotinsäure, sind z.B. in Meyler's Side Effects of Drugs, 10. Auflage, 1984, Elsevier Amsterdam - New York - Oxford aufgeführt.

Wegen ihrer in den Lipiden kompartimentierten Schutzwirkung eignen sich die erfindungsgemäßen lipidlöslichen Antioxidatien vorteilhaft zur Vorbeugung und Behandlung von Erkrankungen, bei denen (z.B. radikalische) Oxidationsprozesse im Lipidmilieu eine Rolle spielen, insbesondere für die Vorbeugung und Behandlung der beschriebenen Vorgänge bei Erkrankungen der Gefäßwand. Aufgrund ihrer insbesondere antioxidativen Eigenschaften können die erfindungsgemäßen Stoffe auch bei anderen medizinischen Problemen, bei denen Bioradikale involviert sind, angewendet werden. Dazu zählen beispielsweise Entzündungsprozesse, insbesondere chronische Entzündungen wie Rheuma oder Arthritis, Mangeldurchblutung durch z.B. cerebrale Schädigungen, wie Schlaganfall, und Tod von Nervenzellen (Alzheimer'sche Krankheit), periphere Gefäßkrankheiten, wie Thrombosen und Atherosklerose, aber auch unerwünschte mutagene, zelltoxische und cancerogene Wirkungen durch Licht oder Strahlen bzw. durch Chemikalien, z.B. Krebstherapeutika, wie Adriamycin, ebenso wie Reperfusionsschädigungen, die nach dem Öffnen von Gefäßverschlüssen, aber auch nach Organ- und Gewebetransplantationen, bzw. nach der Überwindung hypoxischer Bedingungen, z.B. in der Neonatalmedizin, auftreten können. Ferner sind die erfindungsgemäßen Verbindungen auch zur Heilung von Leberschädigungen geeignet.

Für die klinisch-therapeutische Anwendung können die erfindungsgemäßen Antioxidantien auch in Form von Prodrugs, z.B. in Form ihrer Salze vorliegen, aus denen sich dann erst in vivo der Wirkstoff bildet. Als Metallkationen können z.B. solche der Alkalimetalle wie Lithium, Natrium und Kalium, und der Erdalkalimetalle wie Magnesium und Calcium, aber auch kationische Formen, deren Metalle, wie Aluminium, Zink und Eisen verwendet werden, gegebenenfalls chelatisiert mit Zitronensäure oder Ethylendiamintetraessigsäure und dergleichen. Aminkationen sind solche von primären, sekundären oder tertiären Aminen wie der Alkylamine, z.B. Mono-, Di- und Trimethyl; bzw. -ethyl-, -propyl-, -isopropyl-, -butyl-, -isobutyl-, -t-butyl-, sowie N(Methylhexyl)-)-, N-Methyl-hexyl-, Benzyl-$\beta$-phenyl-ethylamin, Ethylendiamin, Diethylentriamin, Pyrrolidin, Piperidin, Morpholin, Piperazin, Mono-, Di- und Triethanolamin, Ethyldiethanolamin, N-Butylethanolamin, Tris-(hydroxymethyl)-aminomethan, und dergleichen. Geeignete Aminsalze sind z.B. die des Tryptamins, Cysteins sowie die basischen Aminsalze des Lysins und des Arginins. Geeignete quaternäre Ammoniumkationen sind z.B. das Tetramethylammonium und das Benzyltrimethylammonium. Diese Kationen können auch zur Salzbildung der anionischen Formen der erfindungsgemäßen Verbindungen verwendet werden, wohingegen zur Salzbildung bei den kationischen Formen Chlorid und Fluorid bevorzugt sind.

**Zubereitung von antioxidativen Zusammensetzungen und von Arzneimitteln**

Die erfindungsgemäßen Verbindungen werden den zu schützenden Lipiden in üblicher Weise zugesetzt. Die zugesetzte Menge des erfindungsgemäßen Antioxidans kann in weiten Bereichen schwanken. Wie im experimentellen Teil ausgeführt wird, können insbesondere hoch konzentrierte Antioxidans/Lipid-Lösungen hergestellt werden. Derart stabilisierte Zubereitungen können dann in verschiedenster Weise, z.B. an Luft, prozessiert werden und anschließend wieder verdünnt werden. Nach dem Verdünnen enthalten Kautschuk, Gummi, Plastik, Fette und Öle im allgemeinen bis zu 1 Gewichtsprozent oder mehr der oben beschriebenen Antioxidatien, wenngleich auch ein Zusatz von 0,1 % ausreichend sein kann. Bei Fetten und Ölen, die der menschlichen Ernährung dienen, werden bis 0,5 Gewichtsprozent, vorzugsweise 0,005 - 0,03 Gewichtsprozent des erfindungsgemäßen Antioxidans angewendet. Die genannten Mischverhältnisse sind auch bei der Herstellung von Lipcsomen anwendbar. Bei der Verwendung als Arzneimittel zur Prophylaxe und zur Behandlung von hyperlipidämischen und thrombotischen peripheren und cerebralen Krankheiten, insbesondere Gefäßkrankheiten bei Mensch und Tier, hängt die erforderliche Dosierung von der Art und Schwere der Erkrankung, bzw. von der zu behandelnden Tierspezies, aber auch von Alter, Gewicht und Gesundheitszustand des Patienten, ab. Bei Menschen kann eine Dosierung von 0,05 mg oder 1 mg bis 100 mg/Tag, insbesondere bei intramuskulärer und intravenöser Dosierung, schon ausreichend sein, wobei aber die Anwendung von bis zu 200 mg oder 500 mg/Tag zu einer höheren Wirkstärke führt. Besonders einfach ist die orale,perorale, rektale oder (trans-)dermale Applikation, die allerdings wesentlich höhere Dosierungen bis über 2,5 g/Tag erforderlich machen kann, wenngleich in der Regel 50 mg bis 800 mg/Tag ausreichend sind. Die genannten Dosierungen können sowohl als Einmaldosis pro Tag, aber auch zweimal oder dreimal bis achtmal täglich in entsprechend reduzierten Dosiseinheiten verabreicht werden.

Die galenischen Zubereitungen für die genannten Applikationen werden gemäß dem Stand der Technik hergestellt. Die erfindungsgemäßen Wirkstoffe können als Pulver, Gel, Emulsion, Dispersion oder Lösung vorliegen und z.B. tropfen- oder löffelweise portioniert werden, bzw. als Inhalt von Kapseln (einschließlich Mikrokapseln und Liposomen) wobei aber bei Verwendung von Kapseln oder Liposomen auch die Hülle die Funktion des Wirkstoffträgers annehmen kann. Dosiseinheiten in Form von festen Arzneiformen, wie

Tabletten (einschließlich Dragees und Pillen), oder Zäpfchen können nach üblichen Verfahren wie Preß-, Tauch- oder Wirbelbettverfahren, oder Kesseldragierung hergestellt werden und enthalten Träger und andere übliche Hilfsstoffe, wie Gelatine, Agarose, Stärke, z.B. Kartoffel, Mais- oder Weizenstärke, Cellulose, wie Ethylcellulose, Siliziumdioxid, verschiedene Zucker, wie Milchzucker, Magnesiumcarbonat und/oder Kalziumphosphate. Die Dragierlösung besteht gewöhnlich aus Zucker und/oder Stärkesirup und enthält meistens noch Gelatine, Gummi arabicum, Polyvinylpyrrolidon, synthetische Celluloseester, oberflächenaktive Substanzen, Weichmacher, Pigmente und ähnliche Zusätze entsprechend dem Stand der Technik. Zur Herstellung der Arzneiformen kann jedes übliche Fließregulierungs-, Schmier- bzw. Gleitmittel wie Magnesiumstearat und Trennmittel verwendet werden. Auch können die Wirkstoffe beispielsweise an Ionenaustauscher (z.B. Polystyrol-divinyl-benzol-sulfonsäure) gebunden oder an Retardierungsmaterial adsorbiert bzw. im Retardierungsmaterial (z.B. solche auf Cellulose- oder Polystyrolharzbasis, z.B. Hydroxyethylcellulose) eingeschlossen sein. Eine verzögerte Freisetzung der Wirkstoffe kann auch erreicht werden, indem die betreffende Schicht mit üblichen magensaftunlöslichen Überzügen versehen wird.

Die hervorragenden antioxidativen Eigenschaften der erfindungsgemäßen lipophilen Verbindungen sind im experimentellen Teil aufgeführt, insbesondere auch im Vergleich zu Antioxidantien gemäß dem Stand der Technik.

**Herstellungsbeispiele:**

Die folgenden Verbindungen der Formel I wurden hergestellt;
falls in den einzelnen Verbindungsformeln an den C-Atomen nichts bzw. nichts anderes steht, sind die etwaigen freien Valenzen mit Wasserstoffatomen abgesättigt:

**1) N-<3-(6-Hydroxy-2,5,7,8-tetramethylchroman-2-carbamoyl)-propyl>-cholsäureamid**

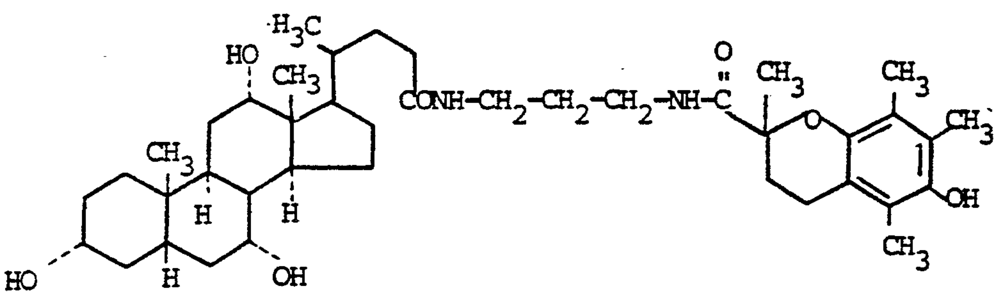

**2) N-<3-(6-Hydroxy-2,5,7,8-tetramethylchroman-2-carbamoyl)-propyl>-desoxycholsäureamid**

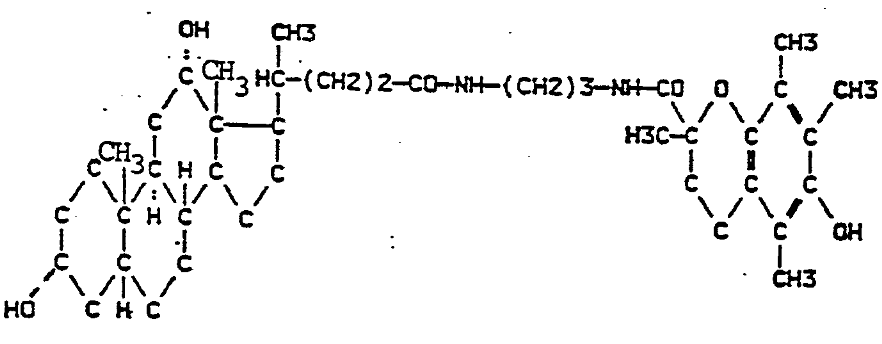

**3) (30)-2-[N-(6-Hydroxy-2,5,7,8-tetramethylchroman-2-carbamoyl)aminoethyl]-3β,7α,12α-cholsäure**

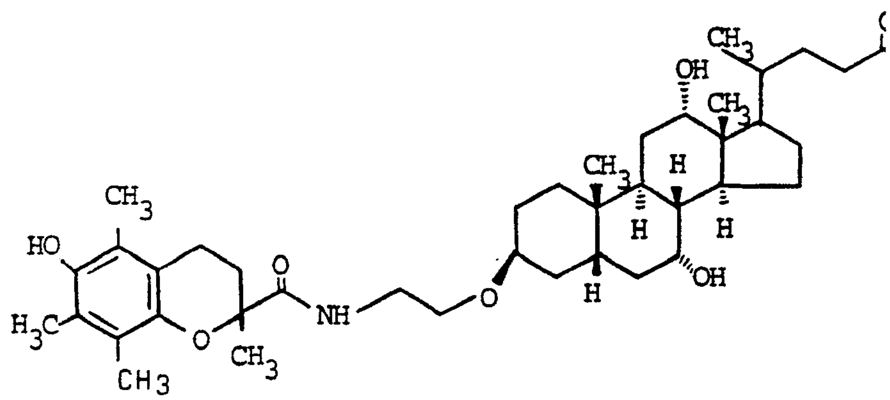

4) N-Hexyl-6-hydroxy-2,5,7,8-tetramethylchroman-2-carboxamid

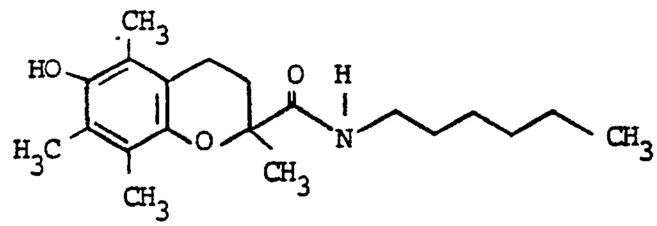

5) N-(3-Heptanamidopropyl)-6-hydroxy-2,5,7,8-tetramethylchroman-2-carboxamid

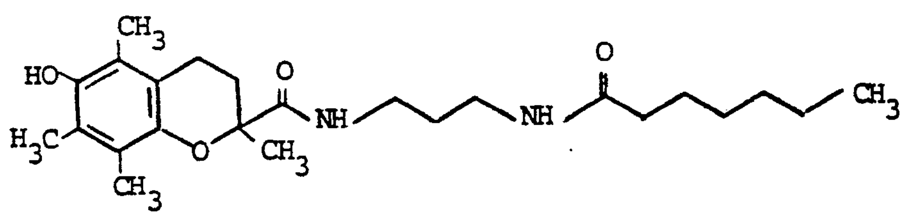

6) N-Octadecyl-6-hydroxy-2,5,7,8-tetramethylchroman-2-carboxamid

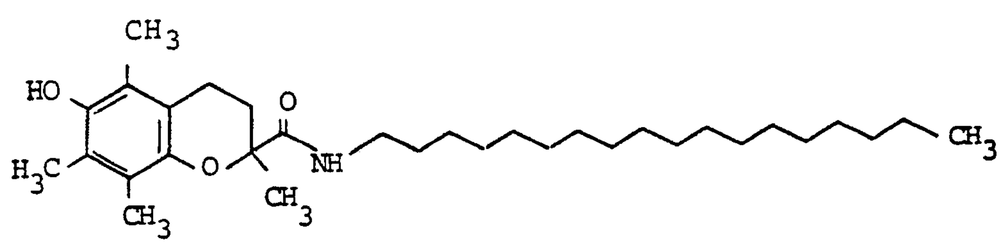

7) N-(3-Hexadecanamidopropyl)-6-hydroxy-2,5,7,8-tetramethylchroman-2-carboxamid

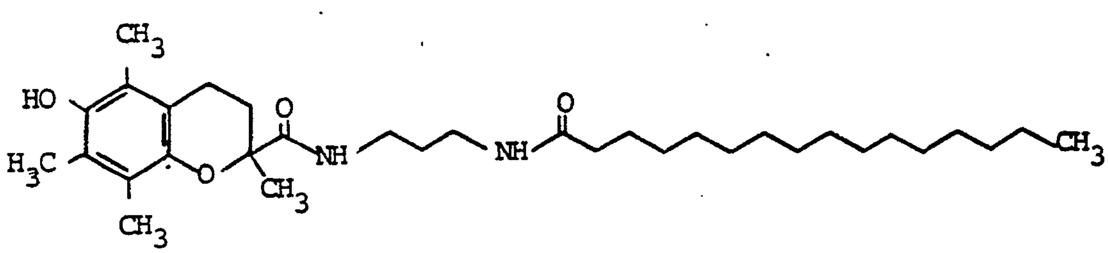

8) Cyclohexyl-6-hydroxy-2,5,7,8-tetramethylchroman-2-carboxamid

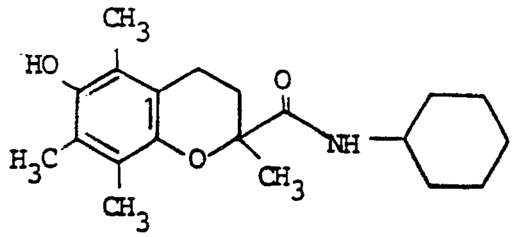

9) N-(3-Octadecanamidopropyl)-4-hydroxy-3-isopropyl-5-tert.-butyl-carboxamid

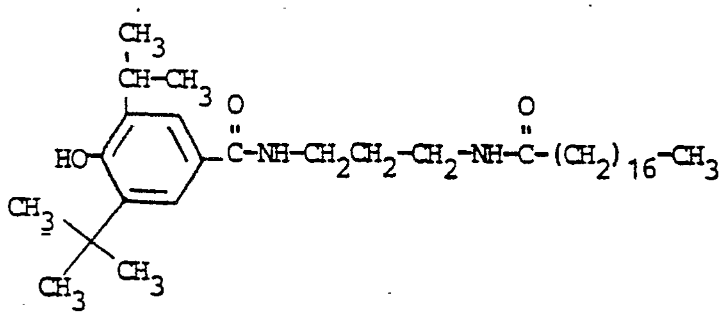

10) 4-[2-(5-Cholesten-3β-yloxy)-ethoxycarbonyl]-2,6-di-tert.-butylphenol

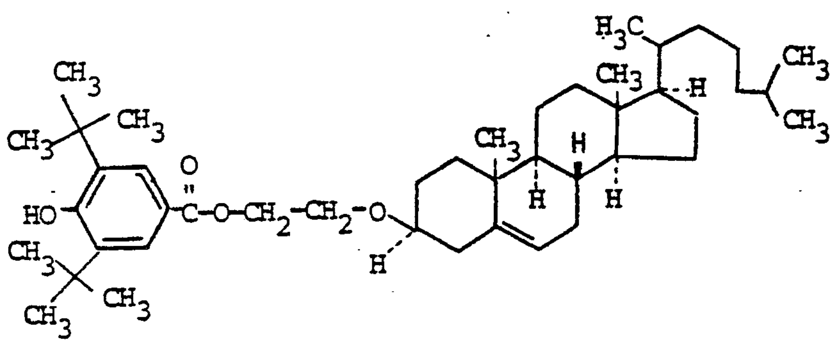

11) 3-[2-(5-Cholesten-3β-yloxy)ethoxycarbonyl]-1,5-dihydroxy-6-methoxy-phenol

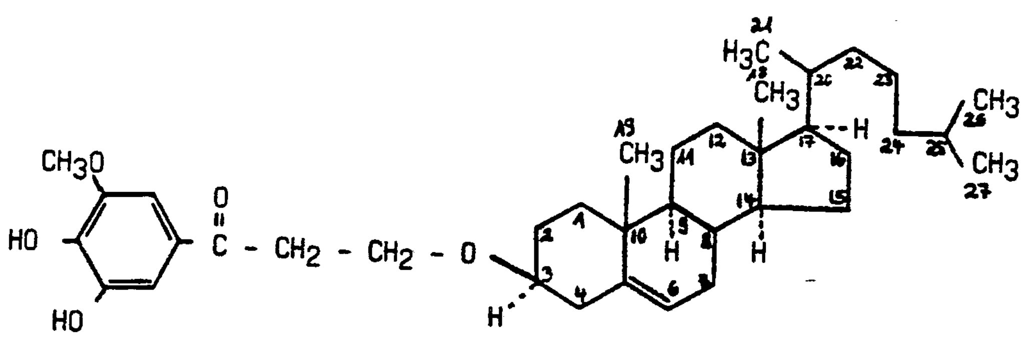

12) 4-[2-(5-Cholesten-3β-yloxy)-ethoxycarbonyl]-2,6-dihydroxy-1-methoxy-phenol

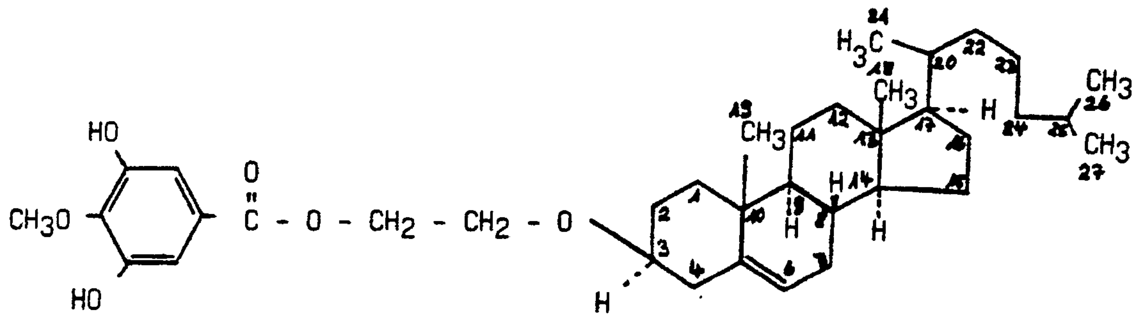

13) 4-<2-(5-Cholesten-3β-yloxy)-ethoxymethyl>-2,6-di-tert.-butylphenol

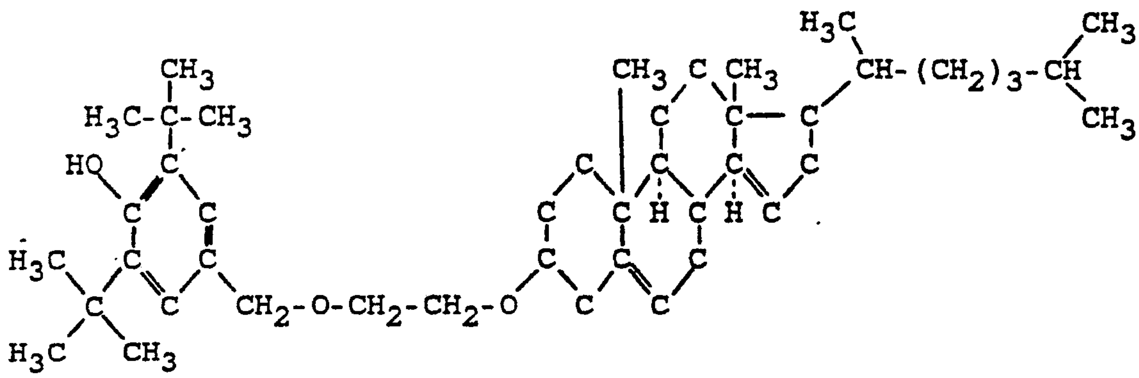

14) 4-[2-(Cholestan-3β-yloxy)-ethoxymethyl]-2,6-di-tert.-butyl-phenol

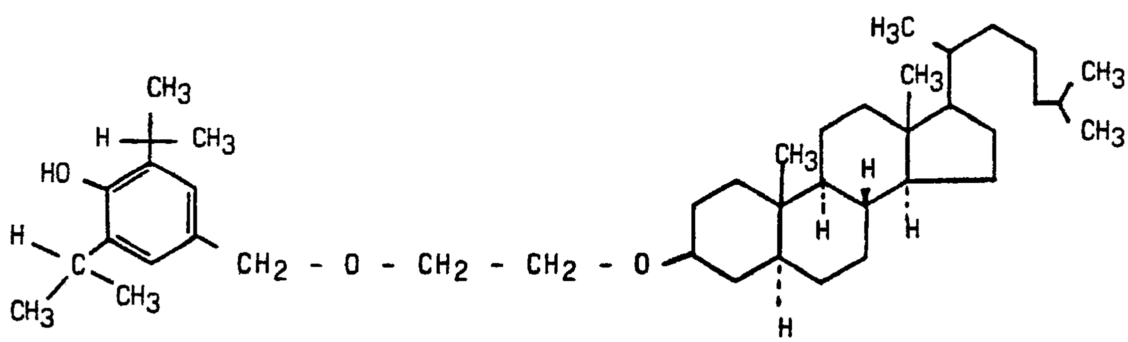

15) 4-[2-(Cholestan-3β-yloxy)-ethoxymethyl]-2-tert.-butyl-6-methyl-phenol

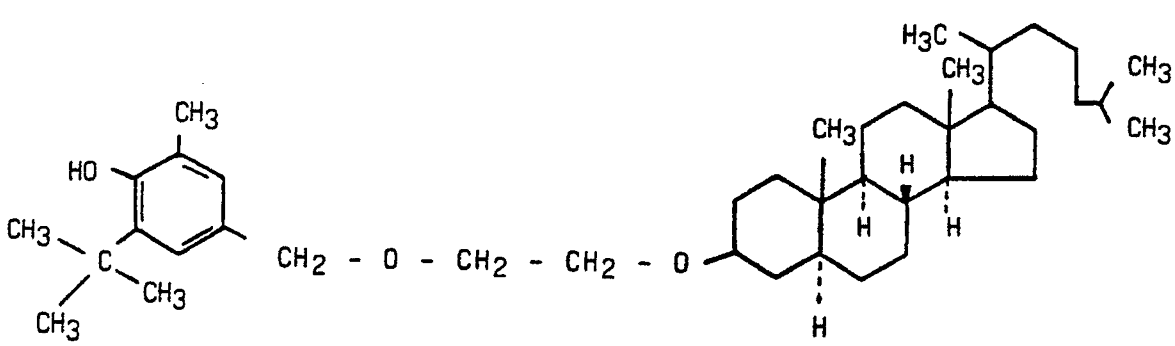

16) 4-[2-(7α,12α-Trihydroxy-5β-cholansäure-3β-yloxy)-ethoxymethyl]-2,6-di-isopropyl-phenol

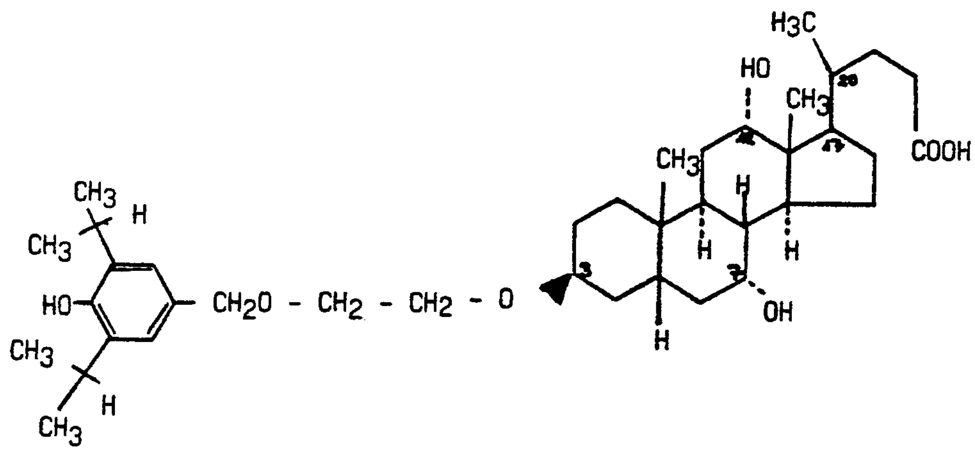

17) 2,6-Di-tert.-butyl-4-<7-noninoyl>-phenol

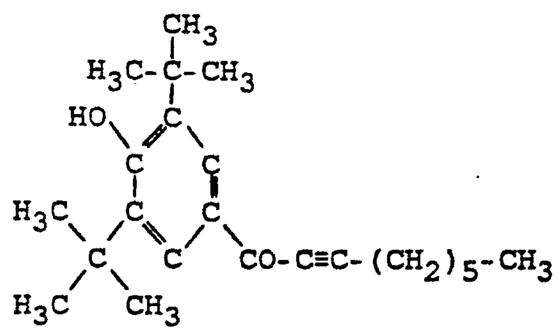

18) 2-(3,5-Di-tert.-butyl-4-hydroxybenzyl)-3-oxo-docosansäure-ethylester

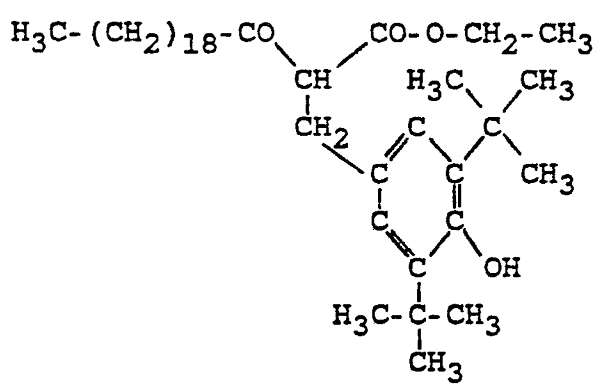

19) 5-[2-(3,5-di-tert.-butyl-4-hydroxyphenyl)-1,3-dithian-4-yl]-valeriansäure

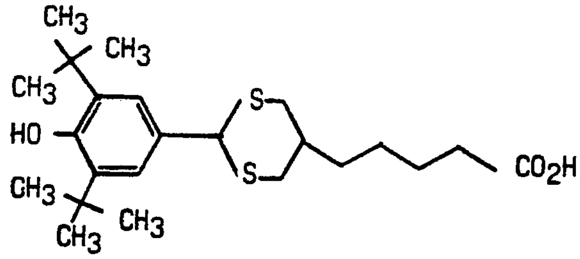

20) 6,8-bis-((3,5-di-tert.-butyl-4-hydroxy-phenyl)-methylthio)-octansäure-tert.-butylester

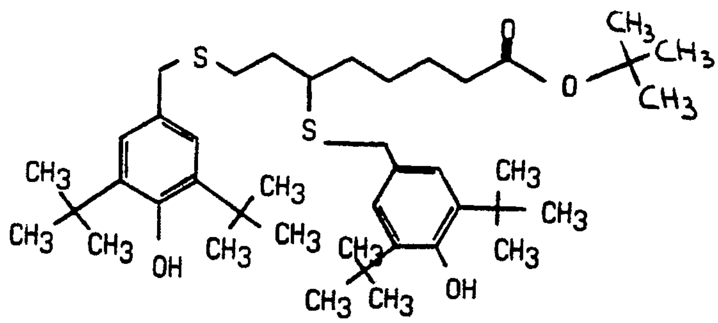

21) (2RS)-1-O-(3,5-di-tert.-butyl-4-hydroxybenzyl)-3-O-octadecylglycerin

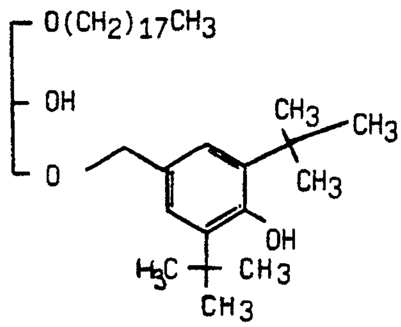

22) (2RS)-2-O-(3,5-di-tert.-butyl-4-hydroxybenzoyl)-1-O-octadecylglycerin

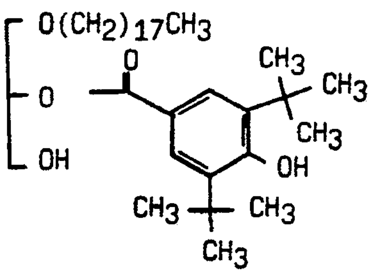

23) (2RS)-1-O-(3,5-di-tert.-butyl-4-hydoxybenzoyl)-3-O-octadecyl-glycerin

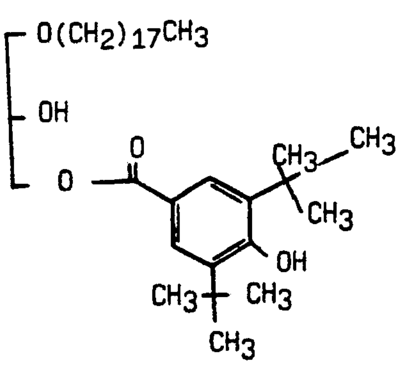

24) 2-(3,5-Di-tert.-butyl-4-hydroxybenzyl)-3-hydroxydocosansäure-ethylester

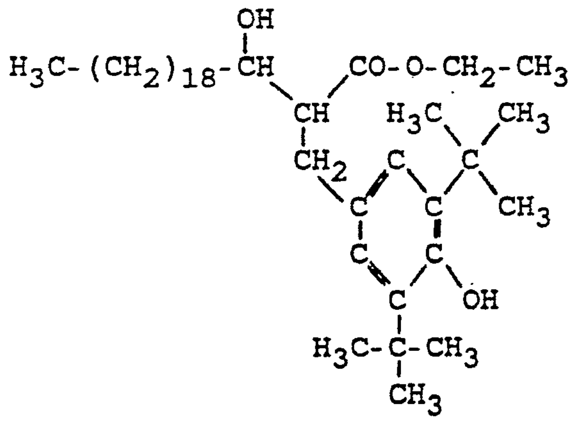

25) 1,3-Dihydroxy-2-(3,5-di-tert-butyl-4-hydroxybenzyl)-docosan

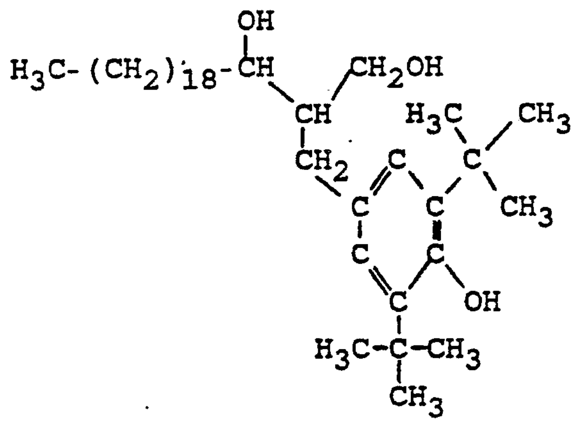

26) 5(RS)-(3,5-Di-tert.-butyl-4-hydroxybenzyl)-2,2-dimethyl-6(R,S)-nonadecyl-1,3-dioxolan

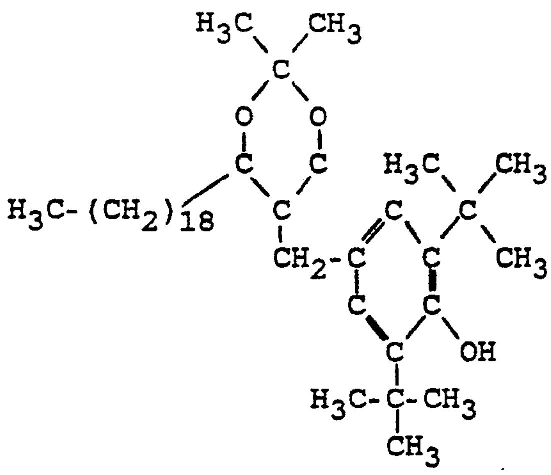

27) 2-(3,5-Di-tert.-butyl-4-hydroxybenzyl)-3-hydroxydocosansäure-ethylester

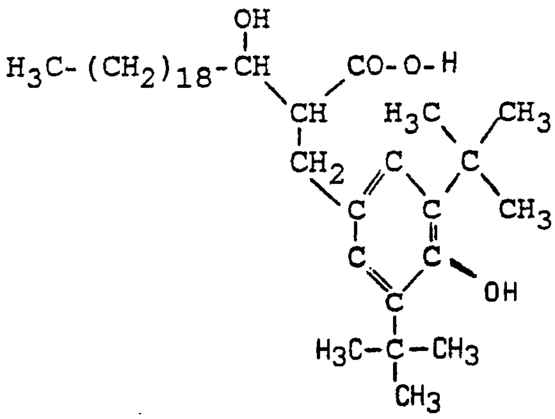

28) (E,Z)-2-(3,5-Di-tert.-butyl-4-hydroxybenzyliden)-3-oxo-docosansäure-ethylester

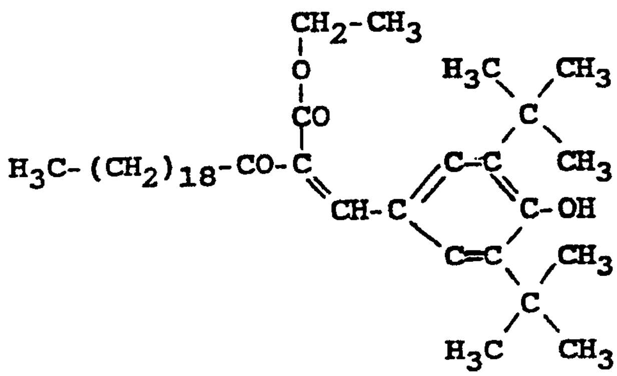

29) 2-O-Octadecyl-3-O-(3,5-di-tert.-butyl-4-hydroxyphenylmethyl)-ascorbinsäure

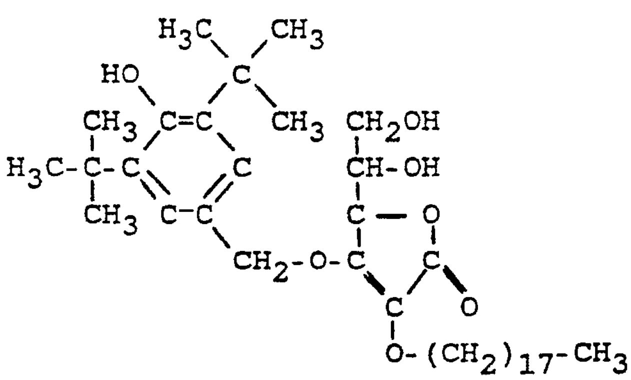

30) 2-Cholersteryloxyethyl-(3'-keto-4',5'-dihydroxy-1',2',6'-trihydrobenzoat)

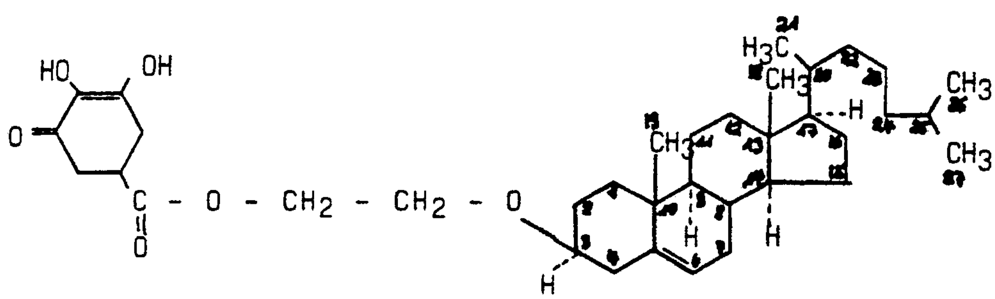

31) 4-Octadecoxy-5-hydroxy-3-keto-4,5-dehydrocyclohexancarbonsäure

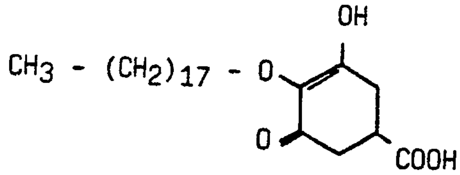

32) Octadecyl-3-keto-4,5-dihydroxy-1,2,6-trihydrobenzoat

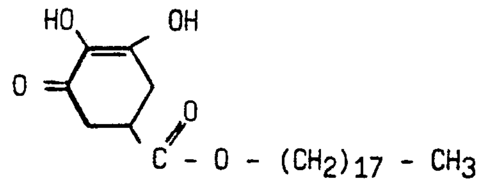

33) 2-O-(2-Cholesteryloxyethyl)-ascorbinsäure

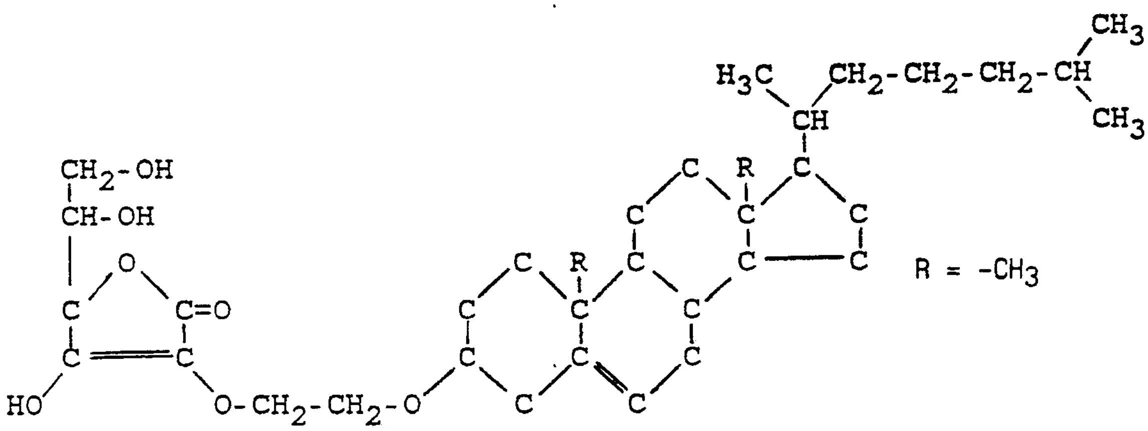

34) 6-O-Octadecanoyl-2-O-(O*,O*-diethylphosphoryl)-ascorbinsäure

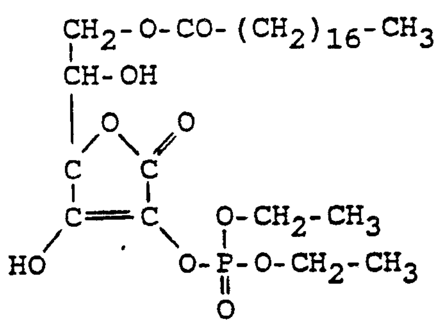

35) 5-O,6-O-Dioctadecanoyl-2-O-(O*,O*-diethylphosphoryl)-ascorbinsäure

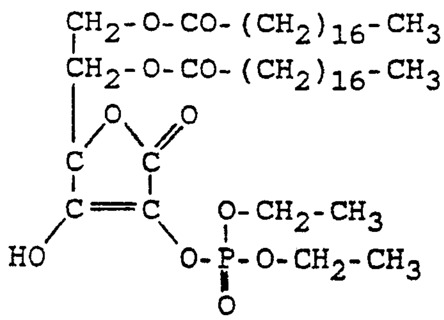

36) 1,3-Bis-<2-(2-O-ascorbyloxy)ethoxy>-2-octadecylpropan

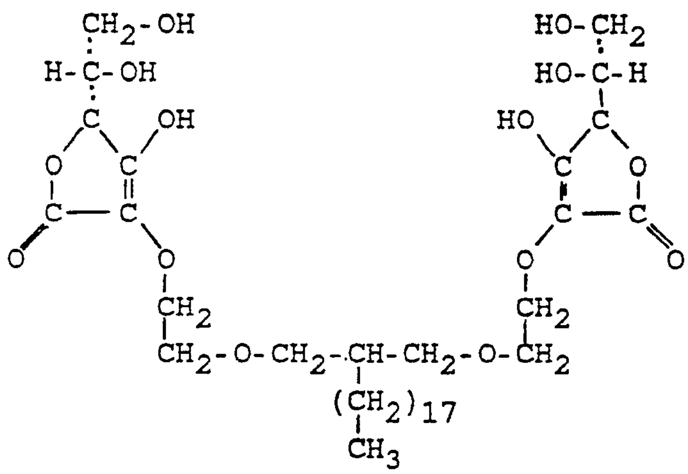

37) Octadecylphosphonsäuredi-(2-O-ascorbyl)ester

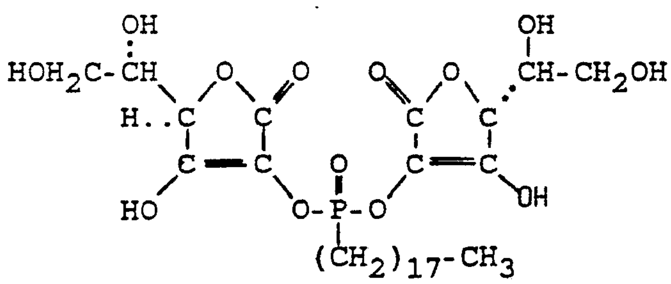

38) 4,5-Dithiacyclohexyl-1,2-distearat

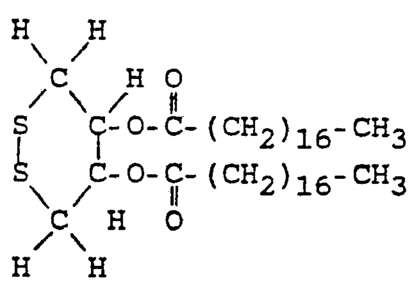

39) 4,5-Dithiacyclohexyl-1,2-distearat, reduziert

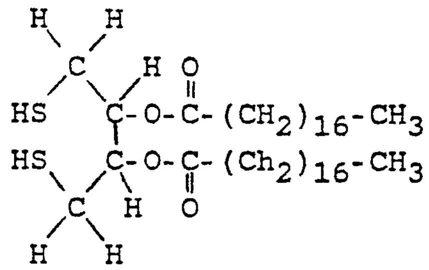

40) 4,5-Dithia-2-hydroxy-cyclohexylstearat

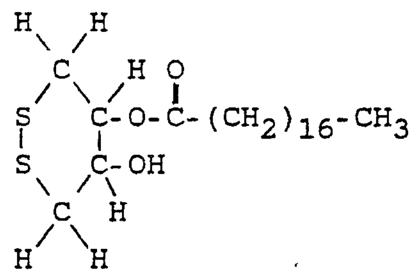

41) 4,5-Dithia-2-hydroxy-cyclohexyl-stearat, reduziert

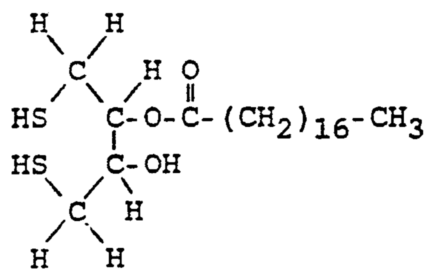

42) 2-Hydroxy-4,5-dithia-cyclohexyl-urso-desoxycholat

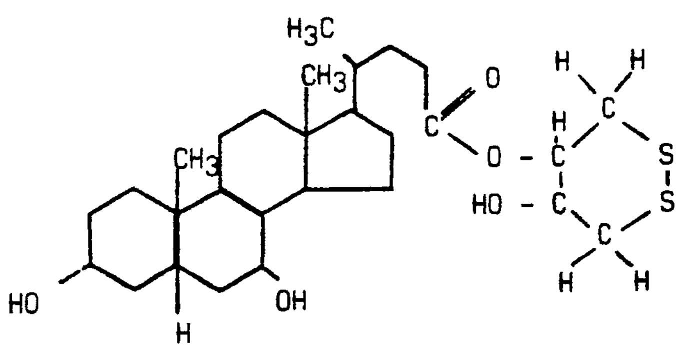

43) 2-Hydroxy-4,5-dithia-cyclohexyl-desoxy-cholat, reduziert

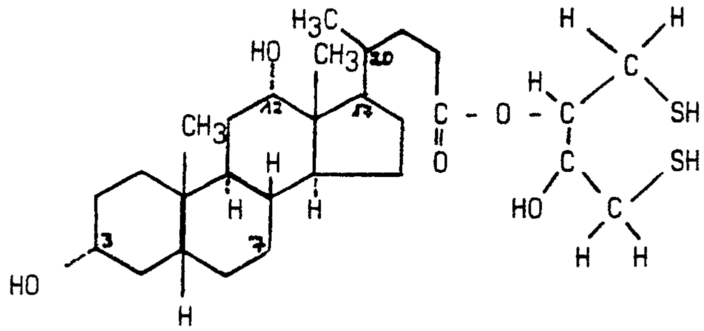

44) Bis-(Cholesterin-6(R,S)-(2',3'-dimercapto-succinat), oxidiert

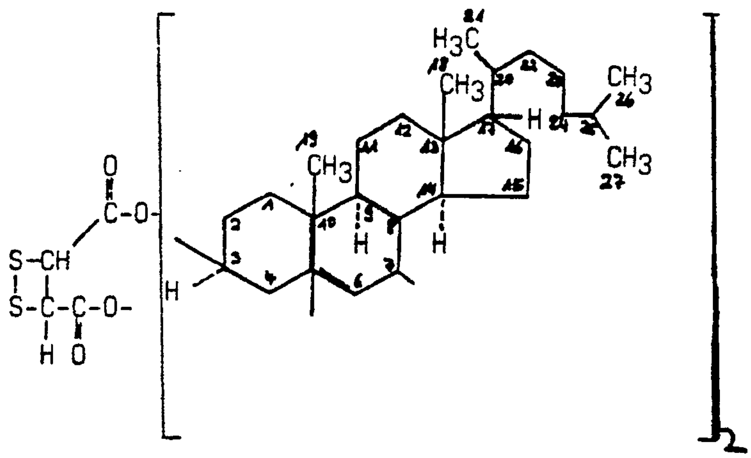

45) Cholesterin-6(R,S)-(2',3'-dimercaptoethylsuccinat)

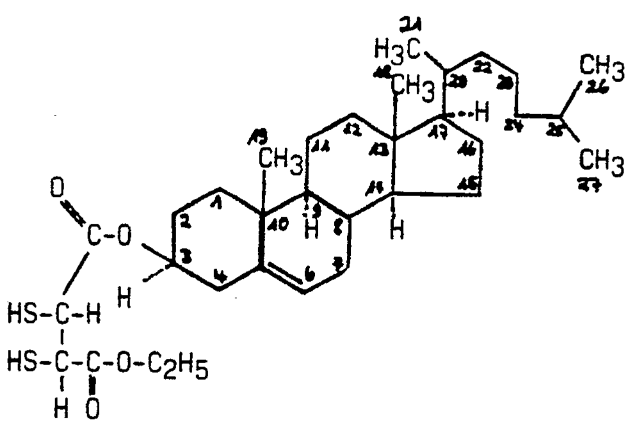

46) Cholesterin-6(R,S)-dihydrolipoat

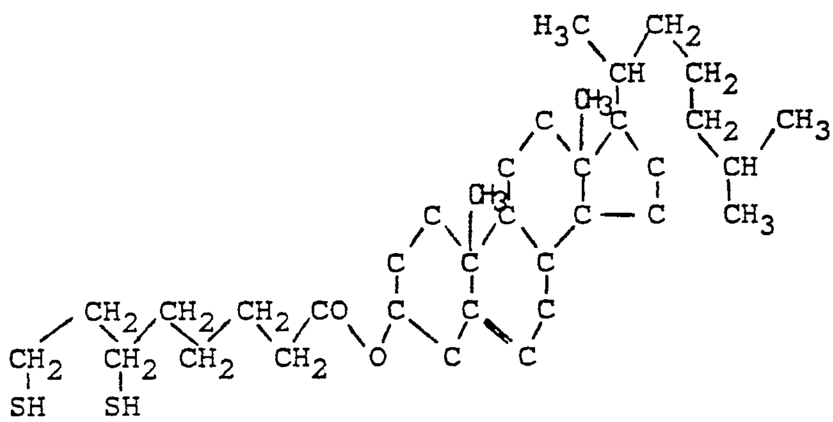

47) N-2-(5-Cholesten-3α-yloxy)ethyldihydroliponsäureamid

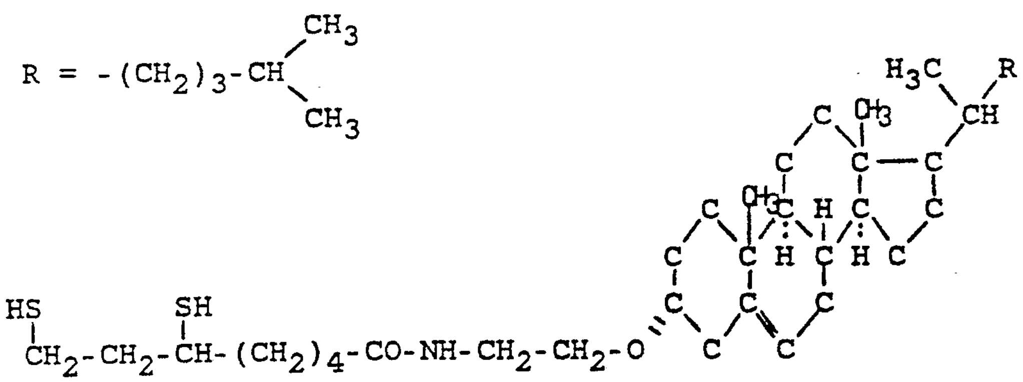

48) N-<3-(6,8-Dimercaptooctanoylamino)propyl>-desoxychosäure

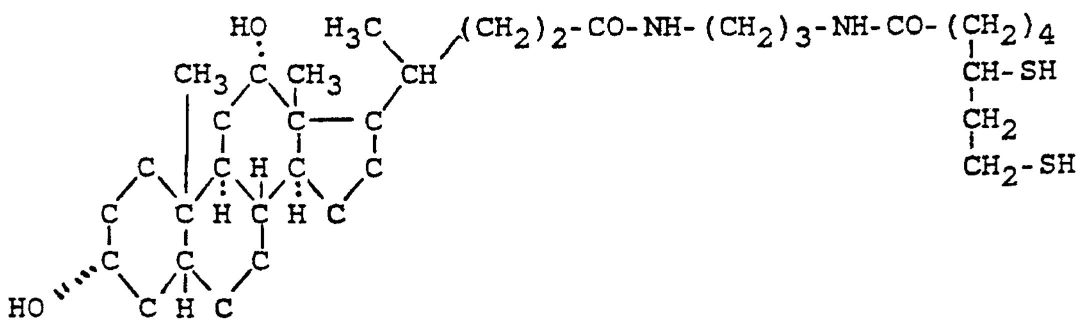

49) N-Octadecyl-DL-dihydroliponsäureamid

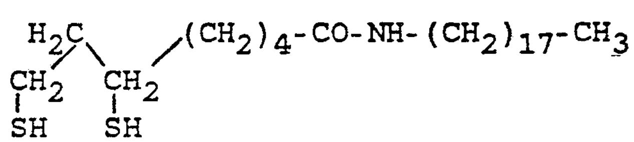

50) DL-Dihydroliponsäure-octadecylester

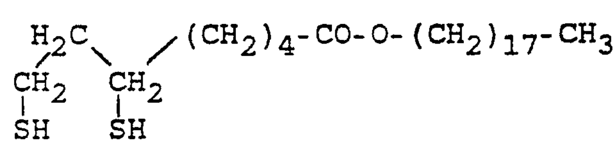

51) DL-α-Liponsäure-octadecylester

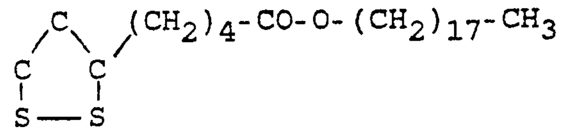

52) N-Octadecyl-DL-α-Liponsäureamid

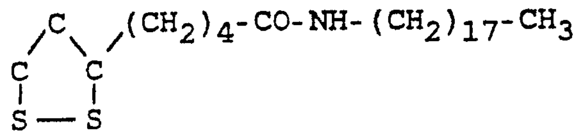

53) Cholesterin-6(R,S)-lipoat

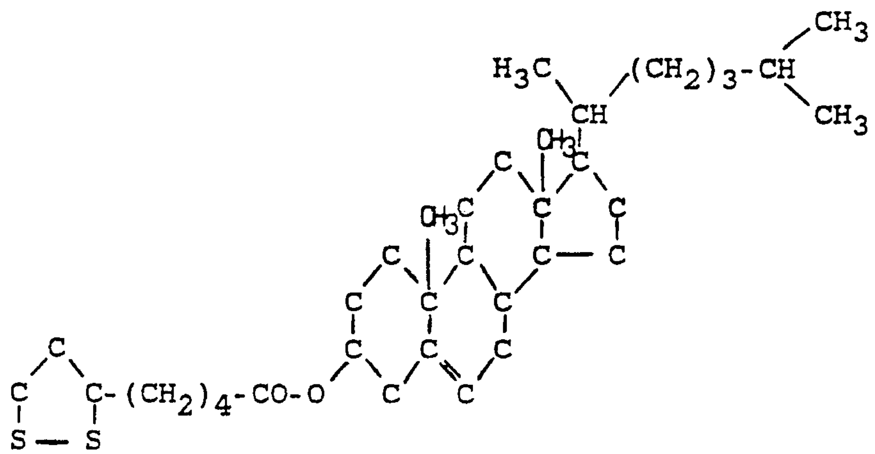

Die Herstellung dieser Verbindungen ist nun im folgenden beschrieben; Ausgangs- und Zwischenprodukte sind mit Nummern ab 70 aufwärts bezeichnet.

**Beispiel 1**

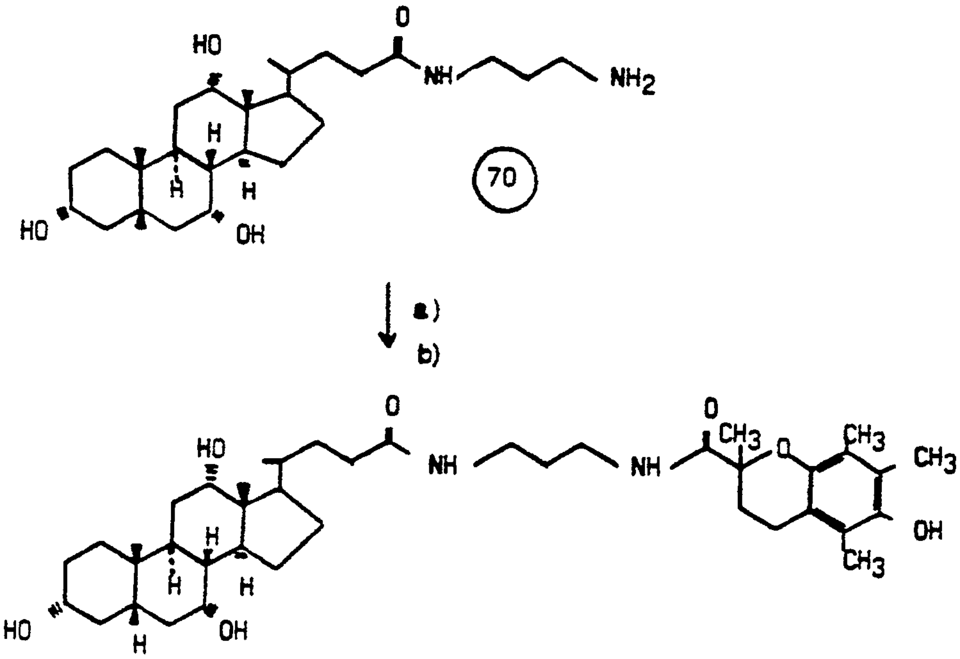


**a)** 1,0 g (4,0 mmol) 6-Hydroxy-2,5,7,8-tetramethyl-chroman-2-carbonsäure (Aldrich) wurde in 50 ml THF/2,8 ml Triethylamin gelöst und bei 0°C mit 0,77 ml (8,0 mmol) Chlorameisensäureethylester versetzt. Es wurde 15 min bei 0°C und 30 min bei Zimmertemperatur gerührt. Anschließend wurden 1,86 g (4,0 mmol) festes Amin 70 [hergestellt durch Umsetzung von Chlorsäuremethylester mit 1,3-Diaminopropan (im Überschuß ohne Lösungsmittel), 5 h, Rückfluß] zugegeben und 3 h bei Zimmertemperatur gerührt. Das Aeaktionsgemisch wurde auf Wasser gegossen, mit Essigester extrahiert (3x) und die vereinigten organischen Phasen getrocknet ($MgSO_4$) und eingedampft. Chromatographie auf Kieselgel (Essigester/Methanol = 10:1) ergab 2,11 g eines weißen Feststoffes. Fp. 102-105°C.

**b)** Zur Freisetzung des Phenols wurden 2,11 g (2,74 mmol) des nach a) erhaltenen Produktes in 50 ml Methanol gelöst und mit 3,8 g (27 mmol) Kaliumcarbonat versetzt. Es wurde 1 h unter Rückfluß erhitzt und das Lösungsmittel weitgehend eingedampft. Der Rückstand wurde mit Wasser ausgerührt und das Produkt abgesaugt. Chromatographie auf Kieselgel ($CH_2Cl_2$/MeOH = 10:1,5) ergab 1,5 g (79 %) Beispiel 2 Fp. 135-140°C.

$C_{41}H_{64}N_{207}$ (696), Ms[1] (FAB[2]), 3-NBA[3]/LiJ): 703 (M[4] + $Li^+$

Dem Fachmann geläufige Abkürzungen:

| | |
|---|---|
| [1] Ms | = Massenspektrum |
| [2] FAB | = Fast Atom Bombardment |
| [3] 3-NBA | = |
| [4] $M^+$ | = Molekülion |

Völlig analog zu Beispiel 1 wurden die Beispiele der Tabelle 1 erhalten.

Tabelle 1

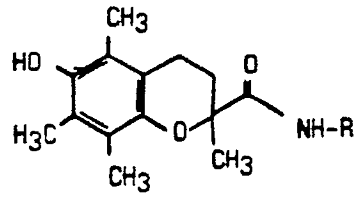

| Beispiel | R | Ms |
|---|---|---|
| 2 | 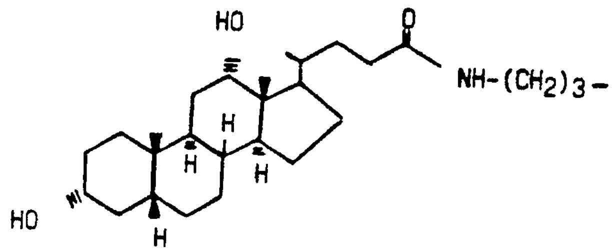 | C41H64N2O6 (608)<br>Ms (FAB): 681 |
| 3 | 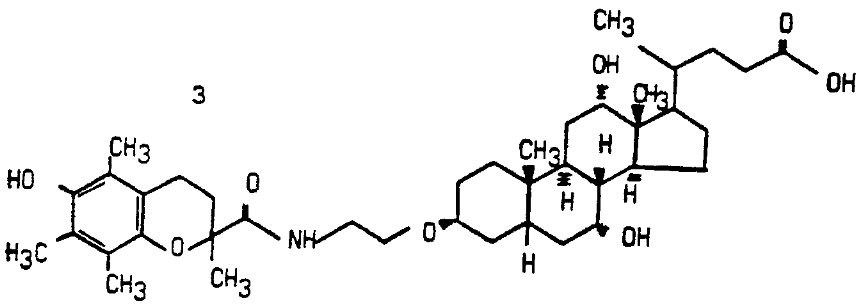 | C40H61NO8 (683)<br>Ms (FAB, 3'NBA/ LiJ): 696 (M+2Li-H), 690 (M+Li) |
| 4 | $-(CH_2)_5-CH_3$ | C20H32NO3 (333)<br>Ms (DCI): 334 (M+H) |
| 5 | $-(CH_2)_3NH-\overset{O}{\overset{\Vert}{C}}-(CH_2)_5-CH_3$ | C24H38N2O4 (418)<br>Ms (FAB): 419 |

Fortsetzung Tabelle 1

| Beispiel | R | Ms |
|---|---|---|
| 6 | $-(CH_2)_{17}-CH_3$ | C32H55O3N (501)<br>Ms (DCI): 502 |
| 7 | $(CH_2)_3NH-\overset{O}{\overset{\|}{C}}-(CH_2)_{14}-CH_3$ | C33H56N2O4 (544)<br>Ms (DCI): 545 |
| 8 | 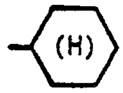 | C20H29NO3 (331)<br>Ms (DCI): 332 |

Beispiel 13

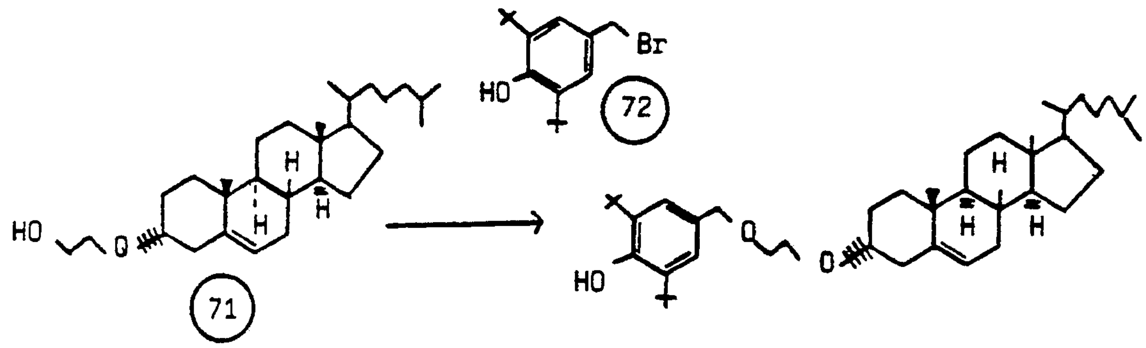
72
71

Zu 61 mg (1,27 mmol) Natriumhydrid in 5 ml THF/5 ml DMF tropfte man 500 mg (1,16 mmol) Steroidalkohol 71 (J. Med. Chem. 1980, 1185) in wenig THF gelöst. Anschließend erwärmte man 30 min auf 50 -60° C. Bei 0° C wurden anschließend 180 mg (0,6 mmol) Bromid 72 fest zugegeben. Nach 1 h bei Raumtemperatur wurden nochmals 80 mg Bromid zugegeben. Man ließ sich nach 1 h bei Raumtemperatur rühren, goß auf Wasser, säuerte die wäßrige Phase mit 1n HCl an und extrahierte mit Ether (3x). Die vereinigten Etherphasen wurden mit ges. $NaHCO_3$ Lösung gewaschen und getrocknet ($MgSO_4$). Eindampfen und Chromatographie auf Kieselgel (Cyclohexan/Essigester = 9:1) gab nach Kristallisation aus Methanol 240 mg Beispiel 13. Fp. 108-110° C.

C44H72O3 (648), Ms (FAB, 3-NBA/LiJ): 655 ($M+Li^+$)

In Analogie zu Beispiel 13 wurden die Beispiele der Tabelle 2 durch Alkylierung der entsprechenden Alkohole (Darst. s. unten) mit dem Bromid 72 erhalten.

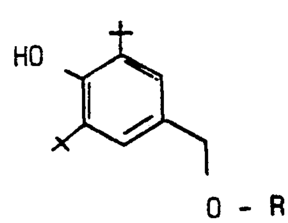

Tabelle 2

| Beispiel | R | Ms |
|---|---|---|
| 21 | 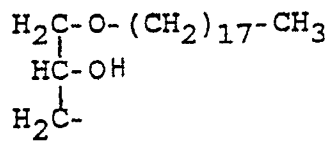 | C36H6604 (562) Ms (DCI): 563 $(M+H^+)$ |

Ausgangsmaterial: Helv. Chimica Acta 71, 274 (1988)

| | | |
|---|---|---|
| 29 | 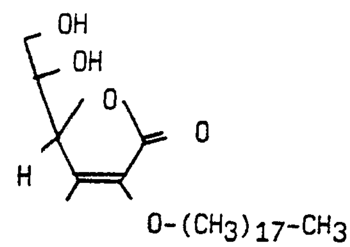 | C39H6507 (645) Ms (DCI): 646 $(M+H^+)$ |

Ausgangsmaterial: Vgl. J. Med. Chem. 31, 793 (1988)

**Beispiel 20**

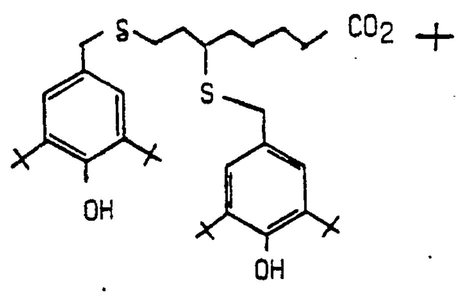

In gleicher Weise wurde Beispiel 20 aus Dihydroliponsäure-t-butylester und 2 Äquivalenten Bromid 72 erhalten. Dihydroliponsäure-t-butylester wurde nach Beispiel 46-50 gewonnen, Liponsäure-t-butylester nach Beispiel 53 und 51.
G42H68S2O04 (700), Ms (DCI): 701 $(M+H^+)$

**Beispiel 17**

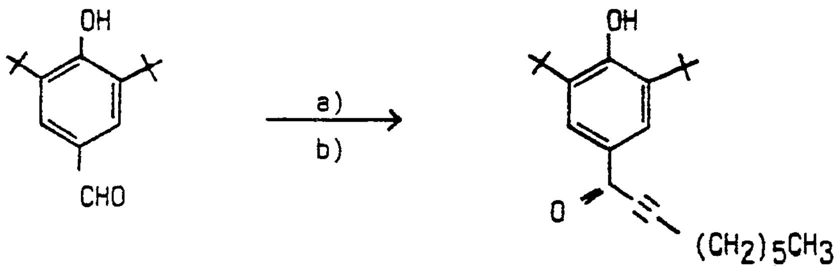


**a)** Zu einer Lösung von 5 ml (33,8 mmol) n-Octin wurden unter Argonatmosphäre zwischen -20 °C und -40 °C 21 ml (33,6 mmol) n-BuLi (Hexan) getropft. Nach 1 h tropfte man 2,8 g (12 mmol) 3,5-Di-tert.-butyl-4-hydroxybenzaldehyd (Aldrich), gelöst in wenig THF, zu. Man ließ über Nacht bei Zimmertemperatur rühren. Das Reaktionsgemisch wurde auf 2n HCl/Eis gegossen und mit Ether extrahiert (3x). Die vereinigten organischen Phasen wurden mit gesättigter Natriumhydrogencarbonatlösung gewaschen (2x) und getrocknet ($MgSO_4$). Eindampfen ergab 4,96 g (quant), das nach b) weiter umgesetzt wurde.
**b)** 4,96 g des nach a) erhaltenen Alkohols wurden in 45 ml Dichlormethan gelöst und mit 4,66 g (21 mmol) Pyridiniumchlorochromat versetzt. Nach 2 h bei Raumtemperatur wurde mit Ether verdünnt und abdekantiert. Filtration über Kieselgel (Cyclohexan/Essigester = 3:1) ergab 2,82 g (57 %) Beispiel 17. Fp. 63-65 °C.

**Beispiel 72**

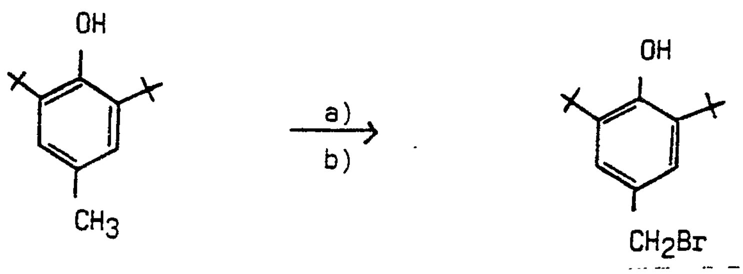


44,8 g (0,2 mol) 2,6-Di-tert.-butyl-p-kresol, 35,6 g (0,2 mol) NBS und 400 mg AIBN wurden in 500 ml Tetrachlorkohlenstoff 2 h unter Rückfluß erhitzt. Nach dem Erkalten wurde abfiltriert und eingedampft. Ausbeute 63,9 g (quantitativ) Beispiel 72.

**Beispiel 18**

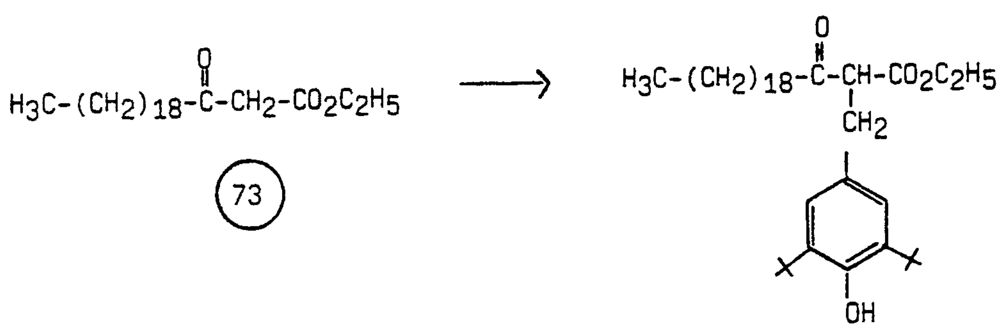

73

Zu 1,09 g (25 mmol) Natriumhydrid in 5 ml THF wurden bei 0 °C unter Stickstoff 3,82 g (10 mmol) Keto-Ester 73 [Keto Ester 73 wurde durch Dianionalkylierung von Acetessigsäurethylester mit Octadecyljodid erhalten. Als Basen wurden NaH und Buli verwendet] in 15 ml THF getropft. Man ließ 30 min bei 0 °C rühren und gab anschließend bei dieser Temperatur 3,0 g (10 mmol) Bromid 72, gelöst in 10 ml THF zu. Nach 2 Tagen bei Raumtemperatur wurde das Reaktionsgemisch auf kalte gesättigte Ammoniumchloridlösung gegossen und mit Ether extrahiert (3x). Die vereinigten organischen Phasen wurden getrocknet

($MgSO_4$) und eingedampft. Präp. HPLC (Cyclohexan/Ethylacetat = 12:1) ergab 3,9 g (65 %) Beispiel 18. C39H68O4 (600), Ms (DCI): 601 ($M+H^+$)

**Beispiel 19**

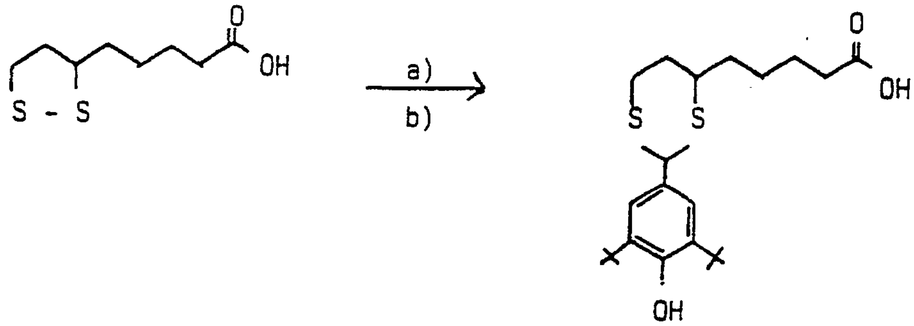


**a)** 100 mg (0,48 mmol) DL-α-Liponsäure wurde in 2 ml 0,25 n wäßrige Natriumhydrogencarbonatlösung gelöst und mit 20 mg Natriumborhydrid versetzt. Man ließ 30 min bei 0 °C rühren, gab 2 ml Toluol zu und stellte mit 2 n Salzsäure auf pH 1. Die organische Phase wurde abgetrennt und eingedampft.

**b)** der nach a) erhaltene Rückstand wurde in 5 ml Dichlormethan aufgenommen und mit 114 mg (0,48 mmol) 3,5-Di-tert.-butyl-4-hydroxybenzaldehyd versetzt. Anschließend gab man 60 µl (0,48 mmol) Bortrifluoridetherat zu und ließ 1 h bei Zimmertemperatur rühren. Das Reaktionsgemisch wurde zwischen Wasser und Essigester verteilt. Die organische Phase wurde abgetrennt, getrocknet ($MgSO_4$) und eingedampft. Chromatographie auf Kieselgel (Cyclohexan/Essigester = 2:1) ergab 135 mg (66 %) Beispiel 19. Fp. 67-68 °C.

C23H36O3S2 (424).

**Beispiel 24**

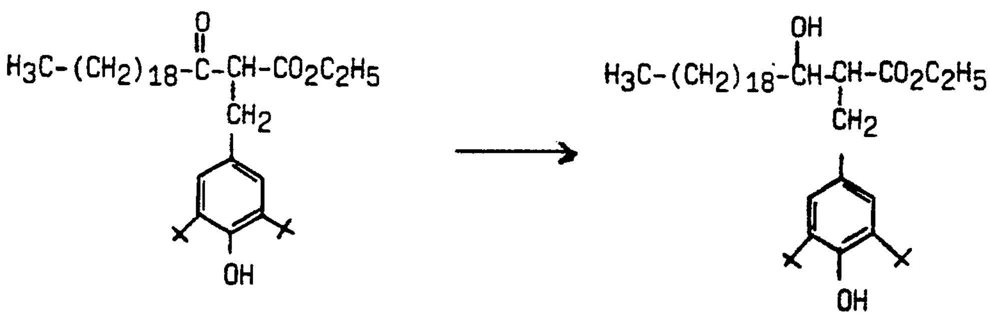

500 mg (0,83 mmol) Beispiel 18 wurden in 15 ml Ethanol gelöst und bei 0 °C mit 112 mg (2,5 mmol) Natriumborhydrid versetzt. Nach 1,5 h Rühren bei 0 °C wurde das Reaktionsgemisch auf 50 ml kalte, gesättigte Ammoniumchloridlösung gegossen und mit Ether extrahiert (3x). Die vereinigten Etherphasen wurden getrocknet ($MgSO_4$) und eingedampft. Chromatographie auf Kieselgel (Cyclohexan/Essigester = 4:1) ergab 480 mg (95 %) Beispiel 24.

C39H70O4 (602), Ms (FAB, 3-NBA/LiJ): 609 ($M+Li^+$)

**Beispiel 25**

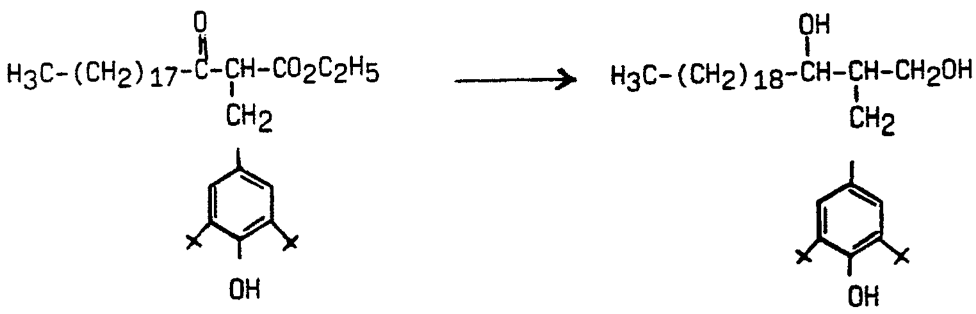

Unter Stickstoff wurden zu 65 mg (1,67 mmol) Lithiumaluminiumhydrid in 10 ml THF bei 0 °C 500 mg (0,83 mmol) Beispiel 18, gelöst in 5 ml THF, getropft. Man ließ 2 h bei Raumtemperatur rühren. Das Reaktionsgemisch wurde auf gesättigte wäßrige Ammoniumchloridlösung gegossen und mit Ether extrahiert (3x). Die vereinigten Etherphasen wurden getrocknet ($MgSO_4$) und eingedampft. Chromatographie auf Kieselgel (Cyclohexan/Essigester = 1:1) ergab 460 mg Beispiel 25. Fp. 77-78 °C.
C37H68O3 (560), Ms (FAB, 3-NBA/LiJ): 567 ($M+Li^+$).

**Beispiel 26**

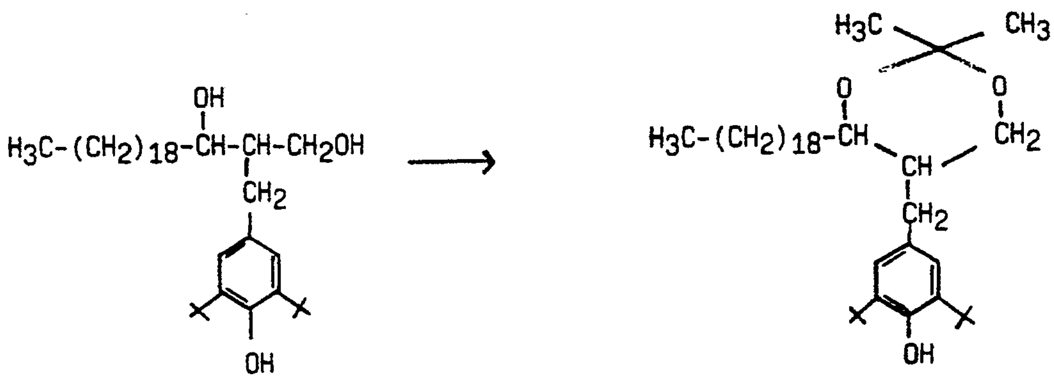

168 mg (0,3 mmol) Beispiel 25 wurden in 10 ml Aceton gelöst und bei Zimmertemperatur mit 0,5 ml Acetylchlorid versetzt. Man ließ 1 h bei Zimmertemperatur rühren, versetzte mit Ether und wusch die Lösung mit gesättigter wäßriger Natriumhydrogencarbonatlösung. Trocknen ($MgSO_4$), Eindampfen und Chromatographie des Rückstandes auf Kieselgel (Cyclohexan/Essigester = 5:1) ergab 164 mg (91 %) Beispiel 26.
C40H72O3 (600), Ms (DCI): 600 (M+).

**Beispiel 27**

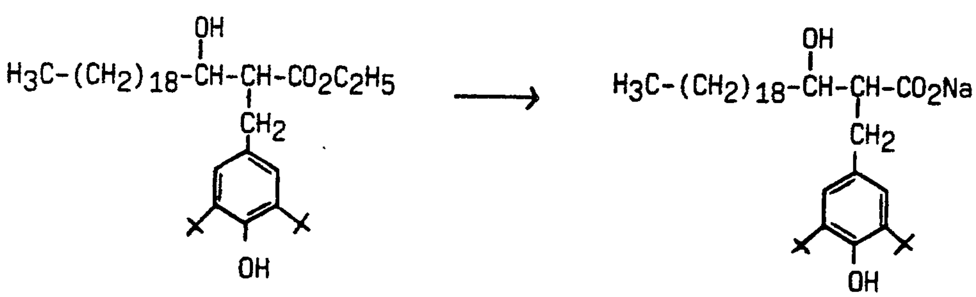

150 mg (0,25 mmol) Beispiel 24 wurden in 5 ml Ethanol gelöst und mit 5,0 ml 0,1 n Natronlauge versetzt. Es wurde 8 h unter Rückfluß gekocht. Das Reaktionsgemisch wurde auf Eis/HCl gegossen und mit Ether extrahiert (3x). Die vereinigten organischen Phasen wurden mit gesättigter Natriumchloridlösung

gewaschen (1x) und getrocknet. Eindampfen ergab die freie Säure. 131 mg der freien Säure wurden in Ethanol gelöst und mit 2,23 ml 0,1 n wäßriger Natronlauge versetzt. Die Lösung wurde mehrmals unter Zusatz von Toluol eingedampft. Man erhielt 130 mg Natriumsalz Beispiel 27.

**Beispiel 28**

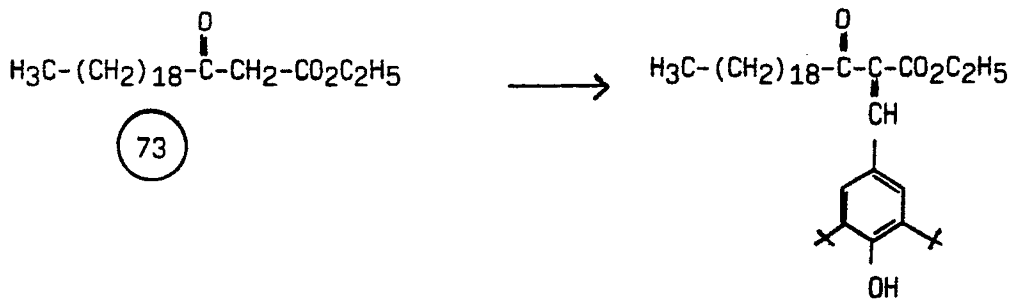

73

1,0 g (2,61 mmol) Keto-Ester 73, 613 mg (2,61 mmol) 3,5-Di-tert.-butyl-4-hydroxybenzaldehyd (Aldrich) wurden in 10 ml Pyridin unter Zusatz von 93 µl (1,2 mmol) Eisessig und 10 µl (0,1 mmol) Piperidin 3 Tage unter Rückfluß erhitzt. Es wurde mit Toluol verdünnt und mit halbgesättigter Kochsalzlösung gewaschen und getrocknet ($Na_2SO_4$). Eindampfen und Chromatographie auf Kieselgel (Cyclohexan/Essigester = 7:1) ergab 740 mg (47 %) Beispiel 28.
$C_{39}H_{66}O_4$ (598), Ms (DCI): 599 $(M+H^+)$

**Beispiel 33**

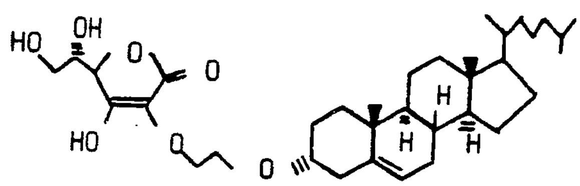

In Analogie zu Beispiel 82,75 (Vorschrift b) und 34 wurde 33 aus Beispiel 74 und 71 hergestellt.
$C_{35}H_{56}O_7$ (588), Ms (FAB): 601 (M+2Li-H), Fp. > 160° (Zers.)

**Beispiel 75**

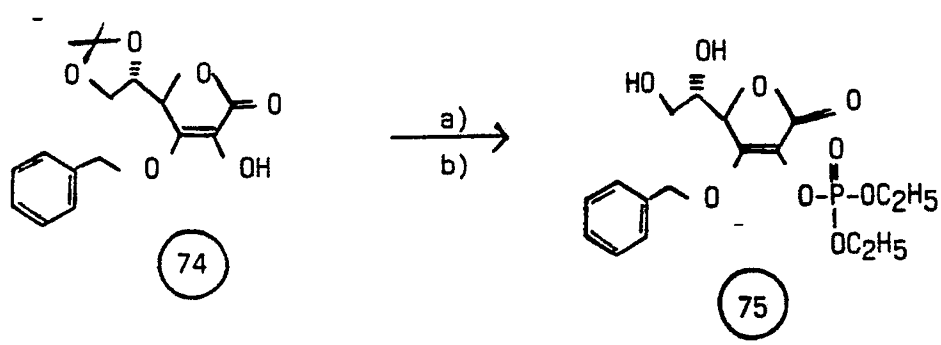


74

75

a) 44,9 g (0,146 mol) Alkohol 74 wurden in 300 ml Dichlormethan gelöst und bei 0°C mit 103 ml (0,733 mol) Triethylamin versetzt. Bei 0°C tropfte man 23,2 ml (0,161 mol) Phosphorsäurediaethylesterchlorid zu und ließ 3 h bei 0°C rühren. Das Reaktionsgemisch wurde auf gesättigte wäßrige Ammoniumchloridlösung gegossen und mit Ether extrahiert (3x). Die vereinigten organischen Phasen wurden getrocknet ($MgSO_4$) und eingedampft. Chromatographie auf Kieselgel (Cyclohexan/Essigester = 3:2) ergab 18,1 g (28 %). Rf (Cyclohexan/Essigester = 1:1) : 0,20.

**b)** Zur Spaltung des Acetonids wurden die nach a) erhaltenen 18,1 g gelöst in 50 ml Ethanol zu 170 ml ethanolischer HCl [hergestellt durch Zutropfen von 3,0 ml Acetylchlorid zu 167 ml Ethanol] gegeben und 2 h unter Rückfluß erhitzt. Das Lösungsmittel wurde eingedampft und der Rückstand über Florisil filtriert (Ethylacetat). Nach Eindampfen erhielt man 14,1 g (85 %) Diol 75. Rf (Essigester) : 0,33.

**Beispiel 76 und 77**

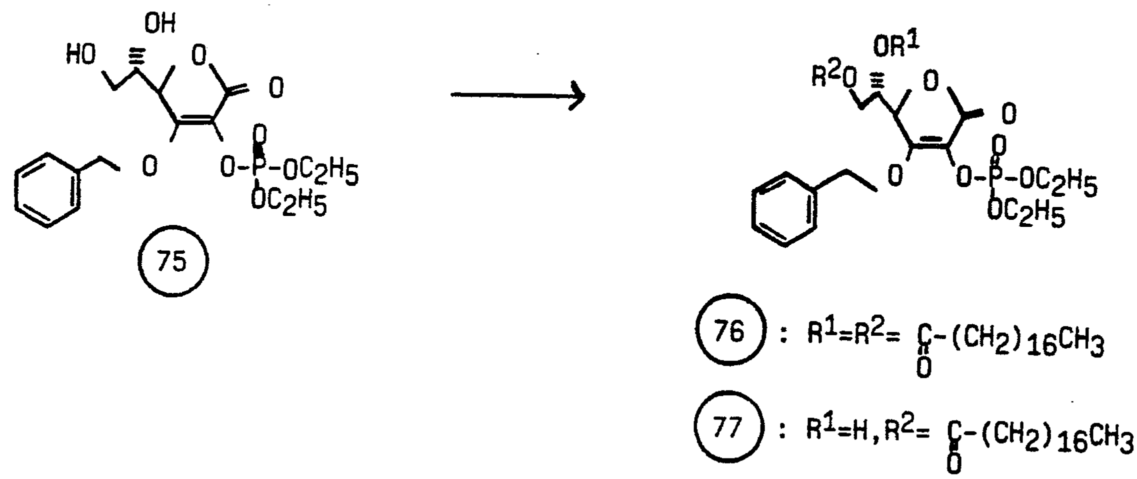


**Beispiel 76**

2,01 g (5 mmol) Diol 75 wurden in 20 ml Pyridin gelöst und bei Raumtemperatur mit 4,8 g (15 mmol) Stearinsäurechlorid versetzt. Man ließ 30 min rühren, goß auf kalte 2 n Salzsäure, und saugte das Produkt ab. Chromatographie auf Kieselgel (Cyclohexan/Essigester = 7:3) ergab 4,11 g (88 %) Beispiel 76.

**Beispiel 77**

Völlig analog zu 76 wurde mit einem Äquivalent Stearinsäure Beispiel 77 erhalten.

**Beispiel 34**

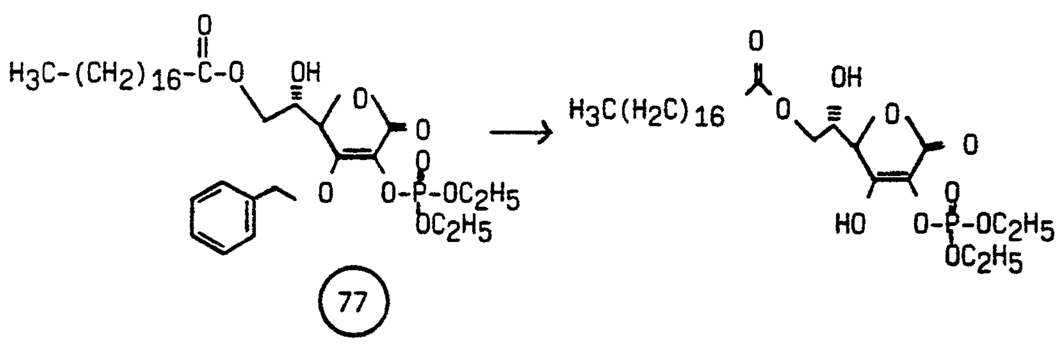


540 mg (0,81 mmol) Beispiel 77 wurden in 10 ml Ethanol mit 100 mg Pd/C (10 %) bei Normaldruck und Zimmertemperatur hydriert. Der Katalysator wurde abfiltriert, das Filtrat eingedampft und der Rückstand mit n-Pentan verrieben. Ausbeute 335 mg (71 %) Beispiel 34. Fp. 84-85° C.
C28H51O10P (578): Ms (FAB, 3-NBA/LiJ): 585 ($M+Li^+$), 591 (M+2Li-H).

**Beispiel 35**

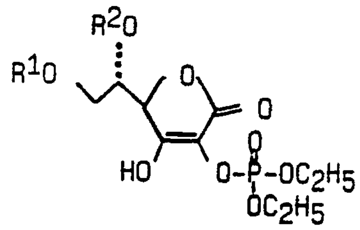

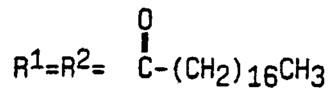

Völlig analog zu Beispiel 34 wurde Beispiel 35 erhalten. Fp. 88-90 °C. C46H85O11 (845), Ms (FAB, 3-NBA/LiJ): 851 (M+Li⁺), 857 (M+2Li-H).

**Beispiel 78**

R = 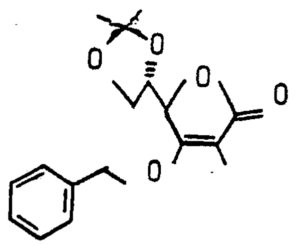

$H_3C-(CH_2)_{17}-CH(-CH_2OCH_2CH_2-OR)_2$

**Ausgangsmaterial**

$H_3C-(CH_2)_{17}-CH(-CH_2OCH_2CH_2OH)_2$ (80)

J. Skarzewski, J. Mlochowski Tetrahedron 39, 309-312 (1983)

**Herstellung**

J. Med. Chem. 31, 793-798 (1988)

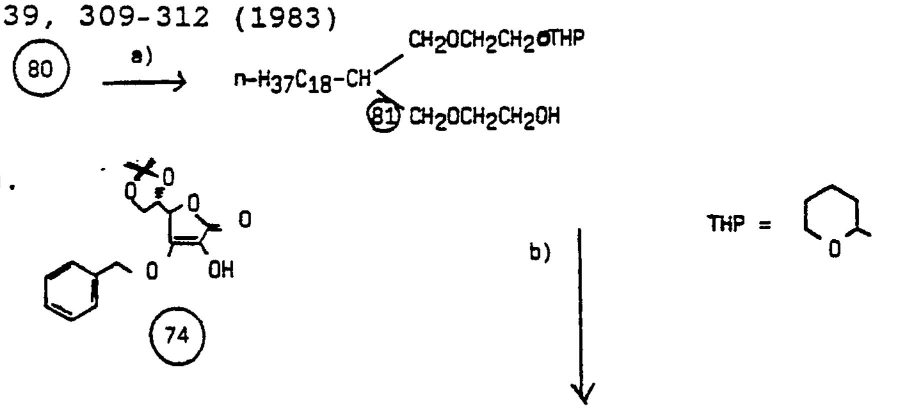


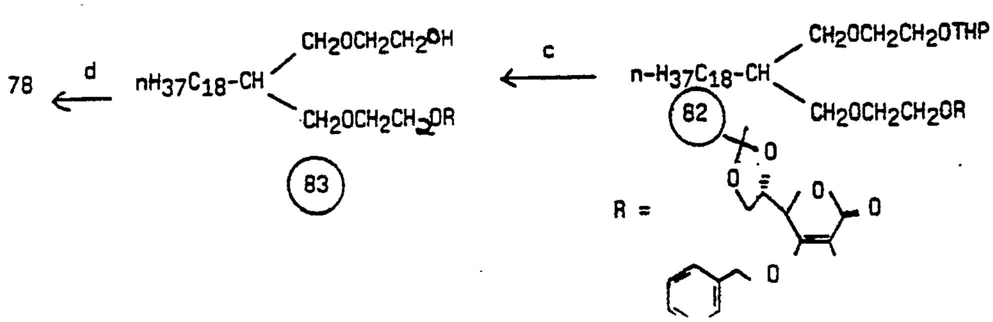

**Beispiel 81 aus 80 a)**

5 g (0,012 mol) Diol 81 wurden in 25 ml Dichlormethan gelöst und mit 1,1 ml (0,012 mol) Dihydropyran und 500 mg Pyridinium-p-toluolsulfonat versetzt. Nach 6 h Rühren bei Raumtemperatur wurde mit 100 ml Ether verdünnt und mit gesättigter Natriumchloridlösung gewaschen (2x). Die organische Phase wurde getrocknet und eingedampft. Chromatographie auf Kieselgel (Essigester/Cyclohexan = 1:1) ergab 3,1 g (52 %) Beispiel 81 neben 1,53 g Bis-THP-Ether und 1,0 g Ausgangsmaterial.

**Beispiel 82 aus 81 b)**

2,75 g (5,5 mmol) Beispiel 81, 1,68 g (5,5 mmol) 74, 1,44 g (5,5 mmol) Triphenylphosphin und 1,1 ml (5,5 mmol) Diisopropylazodicarboxylat wurden in 25 ml THF 1 h bei Raumtemperatur gerührt. Nach dem Eindampfen wurde auf Kieselgel chromatographiert (Essigester/Cyclohexan = 1:2). Ausbeute 2,3 g (53 %) Beispiel 82.

**Beispiel 83 aus 82 c)**

2,2 g (2,8 mmol) Beispiel 82 wurden in 50 ml Ethanol bei Raumtemperatur mit 70 mg Pyridinium-p-Toluolsulfonat versetzt und 4 h bei 50 °C gerührt. Das Reaktionsgemisch wurde eingedampft und der Rückstand zwischen Ether und halbgesättigter NaCl-Lösung verteilt. Trocknen der organischen Phase ($MgSO_4$) und Eindampfen ergab nach Chromatographie auf Kieselgel (Cyclohexan/Essigester = 1:1) 1,3 g (67 %) Beispiel 83.

**Beispiel 78 aus 83(d)**
analog zu Beispiel 82(b).

**Beispiel 79**

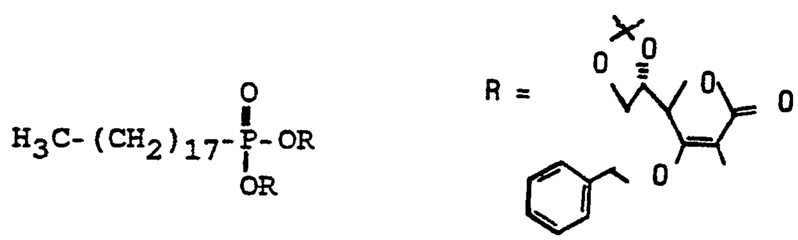
R =

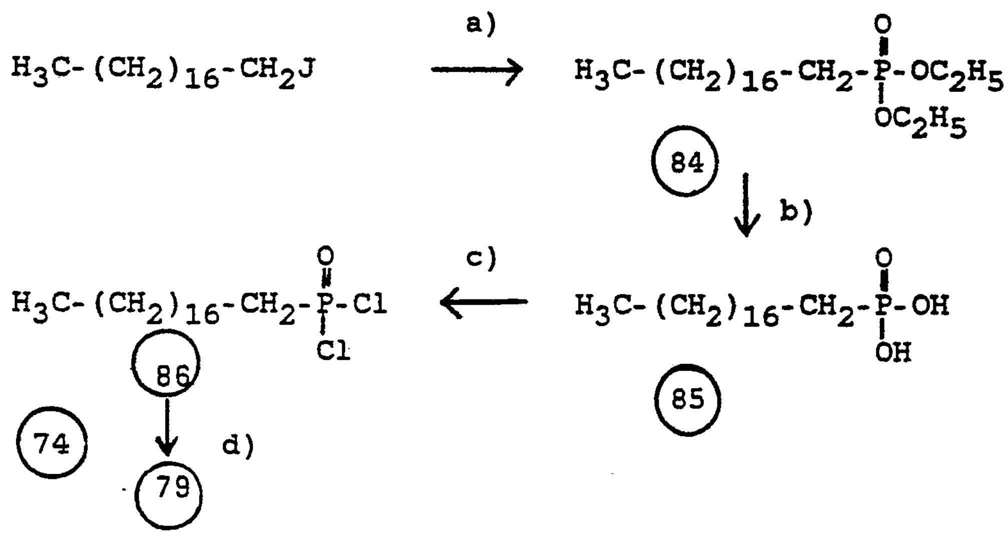


**Beispiel 84 a)**

17,5 g (46 mmol) Octadecyljodid und 8,0 ml (46 mmol) Triethylphosphit wurden 2 h unter Rückfluß gekocht. Chromatographie auf Kieselgel (Essigester) ergab 12,2 g (31 mmol, 68 %) Beispiel 84.

**Beispiel 85 b)**

Die Verseifung von 84 zu 85 wurde durch mehrstündiges Kochen (DC-Kontrolle) mit konzentrierter Salzsäure durchgeführt und in der üblichen Weise aufgearbeitet.

**Beispiel 86 (c)**

1 g Säure Beispiel 85 wurde in 20 ml Thionylchlorid unter Zusatz eines Tropfens DMF 2 h unter Rückfluß gekocht. Eindampfen und mehrmaliges Abrauchen mit Toluol ergab das Säurechlorid 86.

**Beispiel 79 d)**

Die Reaktion wurde analog Beispiel 75 (Vorschrift a) durchgeführt (2 Äquivalente 74). Extraktive Aufarbeitung und Chromatographie auf Kieselgel (Cyclohexan/Essigester = 3:2, 1:1) ergab Beispiel 79. Ausbeute 54 %.

**Beispiel 36 und 37**

In Analogie zu Beispiel 75 (Vorschrift b) und Beispiel 34 erhielt man aus Beispiel 78 Beispiel 36 und aus Beispiel 79 Beispiel 37.

**Beispiel 36**

R = 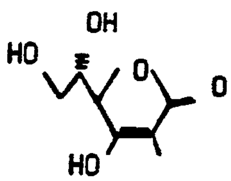

$H_3C-(CH_2)_{17}-CH-(CH_2OCH_2CH_2-OR)^2$
Fp. > 120 °C (Zers.)
C37H64O14 (732), Ms (FAB, 3-NBA/LiJ): 751 (M + 3Li-2H)

**Beispiel 37**

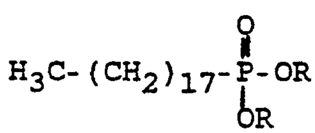

R = 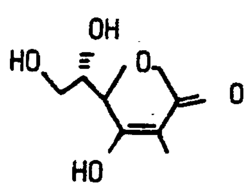

Fp. 72-75 °C
C30H51O13P (650), Ms (DCI): 651 ($M + H^+$)

**Beispiel 46 - 55**

a) Herstellung von Liponsäureestern

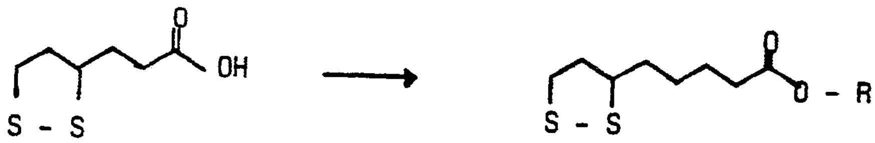

Liponsäure und entsprechender Alkohol werden im molaren Verhältnis von 1:1 in Dichlormethan vorgelegt. Bei Zimmertemperatur gibt man 1 Äquivalent 4-Dimethylaminopyridin und anschließend 1 Äquivalent Dicyclohexylcarbodiimid zu. Man läßt 2-5 h bei Zimmertemperatur rühren, dampft ein, nimmt das Produkt in einem geeigneten Lösungsmittel auf (der Harnstoff bleibt meist größtenteils ungelöst zurück) und chromatographiert auf Kieselgel.

Nach dem Verfahren wurden die in Tabelle 3 aufgeführten Liponsäureester erhalten.

**Tabelle 3**

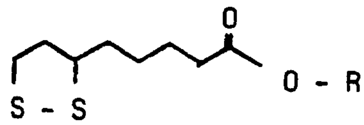

| Beispiel | R | Ms |
|---|---|---|
| 53 | 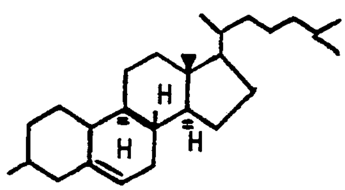 | C35H58O2S2 (574)<br>Ms (DCI): 575<br>$(M+H^+)$ |
| 51 | $-(CH_2)_{17}CH_3$ | C26H50O2S2 (458)<br>Ms (DCI): 458 |

**b)** Herstellung von Liponsäureamiden

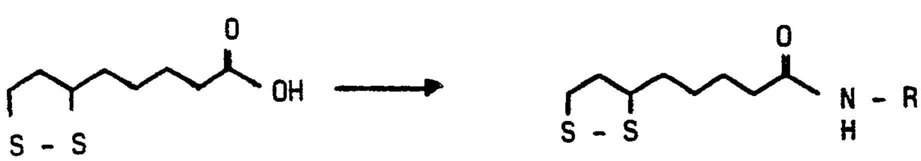

Liponsäure und entsprechendes Amin werden im molaren Verhältnis von 2:1 oder 1:1 in Dichlormethan vorgelegt. Bei Zimmertemperatur gibt man 1 Äquivalent 4-Dimethylaminopyridin und anschließend 1 Äquivalent Dicyclohexylcarbodiimid zu. Man läßt 2-5 h bei Zimmertemperatur rühren, dampft ein, nimmt das Produkt in einem geeigneten Lösungsmittel auf (der Harnstoff bleibt meist größtenteils ungelöst zurück) und chromatographiert auf Kieselgel.

Nach diesem Verfahren wurden die in Tabelle 4 aufgeführten Liponsäureamide erhalten.

**Tabelle 4**

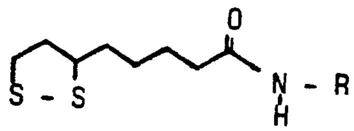

| Beispiel | R | Ms |
|---|---|---|
| 54 | 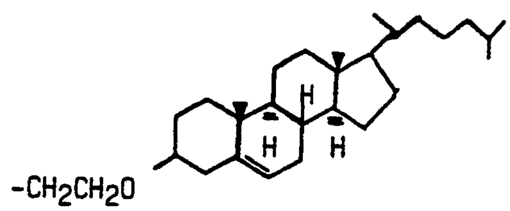 | C37H63NO2S2 (618) Ms (FAB, 3-NBH/LiJ): 624 ($M+Li^+$) |
| (Ausgangsamin durch Gabriel-Synthese aus 71 über das entsprechende Jodid) | | |
| 55 | 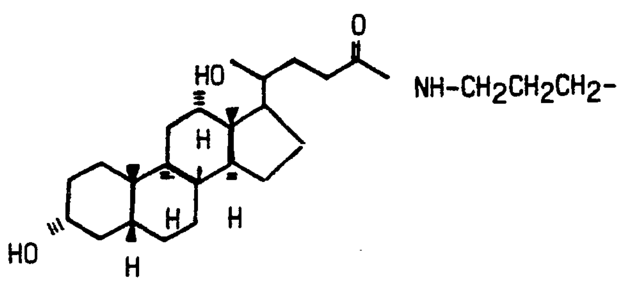 | siehe 48 |
| 52 | $-(CH_2)_{17}CH_3$ | C26H51NOS2 (457) Ms (DCI): 458 ($M+H^+$) |

c) Reduktion von Liponsäurederivaten zu Dihydroliponsäurederivaten

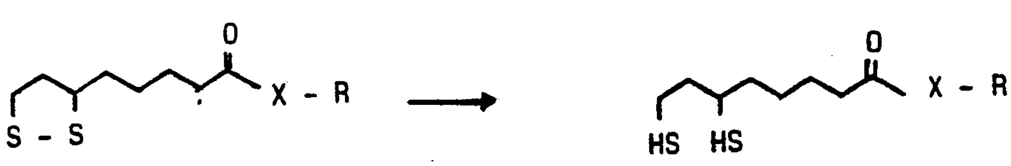

X = O, NH

1 Teil Liponsäurederivat wird in Methanol/THF-Gemischen bevorzugt 1:2) vorgelegt und unter Stickstoffatmosphäre bei 0° C mit 2-3 Äquivalenten Natriumborhydrid versetzt. Nach 2-3 h Rühren bei 0° C wird auf halbgesättigte wäßrige Ammoniumchloridlösung gegossen und mit Essigester extrahiert (2x). Die vereinigten organischen Phasen werden getrocknet ($MgSO_4$) und eingedampft. Das Produkt wird im Hochvakuum getrocknet.

Nach diesem Verfahren wurden die Beispiele der Tabelle 5 hergestellt.

**Tabelle 5**

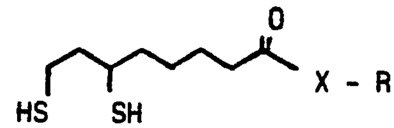

| Beispiel | R | Ms |
|---|---|---|
| | X = O | |
| 46 | 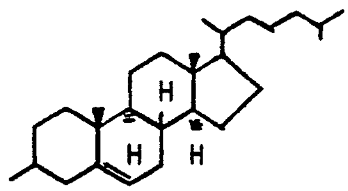 | C35H60O2S2 (576) Ms (FAB, 3-NBA/ LiJ): 583 $(M+H^+)$ |
| 50 | $-(CH_2)_{17}CH_3$ | C26H52O2S2 (460) Ms (DCI): 461 $(M+H^+)$ |
| | X = NH | |
| 47 | 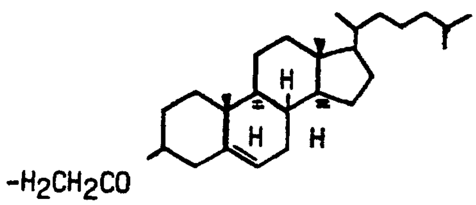 | C37H65NO2 (619) Ms (FAB, 3-NBA/ LiJ): 626 (M+Li) |

**Fortsetzung Tabelle 5**

| Beispiel | R | Ms |
|---|---|---|
| 48 | 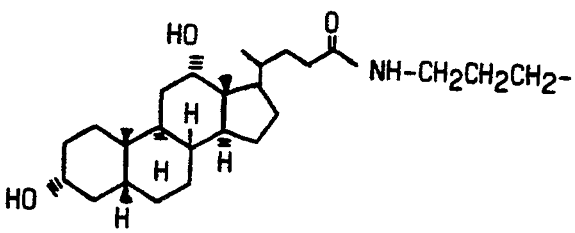 | C35H62N2O4S2 (638) Ms (FAB, 3-NBA/ LiJ): 645 $(M+Li^+)$ |
| 49 | $-(CH_2)_{17}CH_3$ | C26H53NOS2 (459) Ms (DCI): 460 $(M+H^+)$ |

**Lipophile und antioxidative Eigenschaften**

**Methode I**

Antioxidative Radikalfänger-Eigenschaften nach der Diphenylpicrylhydrazin-(DPPH)-Methode:
Die Bestimmung erfolgte spektralphotometrisch (PMQ4 der Fa. Zeiss, Oberkochen, FRG) nach der in Smith und Reeves, Biochemical Pharmacology 36 (1987) S. 1457-1460 beschriebenen Methode. Die antioxidativen Wirkungen der geprüften Präparate sind in Tabelle 1 aufgeführt. Maß ist die in bekannter Weise graphisch (Konzentration versus Umsatzrate) bestimmte Geschwindigkeitskonstante, gemessen in absolutem Ethanol. Mit Ausnahme der oxidierten Mercaptane zeigten alle untersuchten Präparate eine zwar verschieden ausgeprägte, aber deutliche antioxidative Wirkung.

**Methode II**

Verwendung als Zusatz bei Bratfett:
Proben handelsüblicher Deutscher Markenbutter wurden geschmolzen und jeweils mit 1 % (Gewicht/Gewicht) BHT (= butyliertes Hydroxytoluol) bzw. 2,6-Di-tert.-butyl-4-(7-noninoyl)-phenol [= Verbindung gemäß Beispiel 17] bzw. 2-(3,5-Di-tert.-butyl-4-hydroxybenzyl)3-oxo-docosansäureethylester[= Verbindung gemäß Beispiel 18] bzw. N-Octadecyl-DL-α-liponsäuramid [= Verbindung gemäß Beispiel 52], versetzt und in üblicher Weise als Bratfett verwendet. Nach dem Bratvorgang war die mit BHT versetzte Probe in eine zähflüssige, dunkelbraune Masse übergegangen, während der Einsatz der genannten Vergleichspräparate zu einer wesentlich geringeren Farbveränderung führte. Die bessere Schutzwirkung der Vergleichspräparate kommt vermutlich aufgrund ihrer lipophilen Seitenketten und deshalb verbesserter lipophiler Wechselwirkung zustande. Besonders überraschend ist die vorteilhafte Wirkung von N-Octadecyl-DL-α-liponsäureamid, obwohl dieses Präparat keine erkennbare antioxidative Komponente besitzt.

**Methode III**

Lipidlöslichkeit der Verbindungen und Schutzwirkung.
**a)** Herstellung von Olivenöl- bzw. wäßrigen Lösungen. 1 mg bzw. 10 mg bzw. 50 mg der jeweiligen Verbindung wurden bei 37° C mit 1 ml Olivenöl bzw. mit 1 ml bidestilliertem Wasser (bzw. verdünnter NaOH pH = 7,6) versetzt und überprüft, ob eine klare Lösung erhalten wurde. Die gegebenenfalls zentrifugierten und dekantierten Olivenöllösungen wurden 5 Minuten mit dem Bunsenbrenner erhitzt und der Bräunungsgrad des Olivenöls festgestellt. Unter diesen Bedingungen wird das Öl ohne Zusatz und ohne Schutzwirkung deutlich dunkelbraun. Nach Zusatz von Antioxidans und bei guter Schutzwirkung wird das Öl nur dunkelgelb bis hellbraun. Proben mit schlechter Schutzwirkung sind in der Tabelle (s.u.) mit (*) versehen.
Vorteilhaft ist auch die extrem hohe Löslichkeit (>1:1) der erfindungsgemäßen Antioxidantien in geschmolzenem Cholesterolpalmitat bei 85° C.

Tabelle 1: Ergebnis

| Geprüfte Verbindungen: | Methode I Reaktion mit DPPH (Geschwindigkeitskonstante) | Methode III Löslichkeit (mg/ml) Wasser | Olivenöl |
|---|---|---|---|
| Vitamin E-Analoga | | | |
| 6-Hydroxy-2,5,7,8-tetramethylchroman-2-carbonsäure (1) | 2.65 | >10.0 | < 0.1 |
| Vitamin E (1) | 2.90 | < 0.1 | >10.0 |
| Vbg. gem. Bsp. 1 | 0.681 | > 0.1 | >10.0 |
| " " " 2 | 0.528 | > 0.1 | >10.0 |
| " " " 3 | | < 0.1 | > 1.0 |

**Tabelle 1: Ergebnis**

| Geprüfte Verbindungen: | Methode I Reaktion mit DPPH (Geschwindigkeitskonstante) | Methode III Löslichkeit (mg/ml) Wasser | Olivenöl |
|---|---|---|---|
| Vitamin E-Analoga | | | |
| 6-Hydroxy-2,5,7,8-tetramethylchroman-2-carbonsäure (1) | 2.65 | >10.0 | < 0.1 |
| Vitamin E (1) | 2.90 | < 0.1 | >10.0 |
| Vbg. gem. Bsp. 4 | | > 0.1 | >10.0 |
| " " " 5 | | < 0.1 | >10.0 |
| " " " 6 | 0.254 | < 0.1 | >10.0 |
| " " " 7 | 0.355 | < 0.1 | >10.0 |
| " " " 8 | | < 0.1 | >10.0 |
| Phenole | | | |
| Gallussäure (1) | | >10.0 | < 0.1 |
| BHT (1) | | < 0.1 | >10.0 |
| Vbg. gem. Bsp. 13 | 0.061 | < 0.1 | >50.0 |
| " " " 17 | 0.004 | < 0.1 | >50.0 |
| " " " 18 | | < 0.1 | >50.0 |
| " " " 19 | 0.268 | < 0.1 | >50.0 |
| " " " 20 | | < 0.1 | >50.0 |
| " " " 21 | 0.236 | < 0.1 | >50.0 |
| " " " 22 | 0.004 | < 0.1 | >50.0 |
| " " " 23 | 0.006 | < 0.1 | >50.0 |
| " " " 24 | 0.054 | < 0.1 | >50.0 |
| " " " 25 | 0.052 | < 0.1 | >50.0 |
| " " " 26 | 0.065 | < 0.1 | >50.0 |
| " " " 27 | | >10.0 | > 1.0 |
| " " " 28 | | < 0.1 | >50.0 |
| " " " 29 | 0.711 | > 1.0 | >10.0 |

**Tabelle 1: Ergebnis**

| Geprüfte Verbindungen: | Methode I Reaktion mit DPPH (Geschwindigkeitskonstante) | Methode III Löslichkeit (mg/ml) Wasser | Olivenöl |
|---|---|---|---|
| Ascorbinsäure-Analoga | | | |
| Ascorbinsäure (1) | 2.99 | >10.0 | < 0.1 |
| Ascorbylpalmitat (1) | | < 0.1 | > 1.0 |
| Vbg. gem. Bsp. 34 | 0.052 | < 0.1 | > 1.0 |
| " " " 35 | 0.053 | < 0.1 | > 1.0 |
| " " " 37 | 1.13 | >10.0 | > 1.0 |
| " " " 33 | | < 0.1 | > 1.0 |
| " " " 36 | 0.242 | >10.0 | >10.0 |
| Mercaptane | | | |
| Dihydroliponsäure, Na-Salz (1) | 3.147 | >10.0 | < 0.1* |
| Dithiothreitol (1) | 5.632 | >10.0 | < 0.1 |
| Dithioerythrit (1) | | >10.0 | < 0.1 |
| 2,3-Mercaptobernsteinsäure (1) | | >10.0 | < 0.1 |
| Vbg. gem. Bsp. 46 | 0.440 | < 0.1 | >50.0 |
| " " " 47 | 0.075 | < 0.1 | >50.0 |
| " " " 48 | 0.337 | < 0.1 | >50.0 |
| " " " 49 | 0.243 | < 0.1 | >10.0 |
| " " " 50 | 0.248 | < 0.1 | >50.0 |
| Oxid. Mercaptane | | | |
| Liponsäure, Na-Salz (1) | 0.0 | >10.0 | < 0.1* |
| Vbg. gem. Bsp. 51 | 0.0 | < 0.1 | >50.0 |
| " " " 52 | 0.0 | < 0.1 | >10.0 |
| " " " 53 | | < 0.1 | >50.0 |

1) = nicht erfindungsgemäß

**Ergebnis:**

Die erfindungsgemäßen Antioxidantien weisen neben der erforderlichen Lipidlöslichkeit hervorragende antioxidative Schutzwirkungen auf.

Die Befunde der Tabelle 1 wurden ergänzt durch die experimentelle Bestimmung des Verteilungskoeffizienten $K_d$ (Butanol/Wasser-Methode nach Carney und Graham, Arzneim.-Forschung 35 (1985) 228-233), der die Lipophilie der Verbindungen bestätigte.

Experimentell konnten die erfindungsgemäßen Verbindungen auch durch Octanol aus wäßrigen Lösungen bzw. Suspensionen (bestehend aus 1 mg Antioxidans/ml physiologische Kochsalzlösung pH = 7.6) praktisch vollständig (zu ca. 100 %) extrahiert werden.

Die Verbindung gemäß Beispiel 36 ist aufgrund ihrer Löslichkeitseigenschaften hervorragend zur Verwendung in wäßrigen Ölemulsionen geeignet.

**Methode IV**

Inhibierung der Oxidation von humanphysiologischen Lipiden.

Oxidation von 1-Stearoyl-2-arachidonoyl-phosphatidylcholin (SA-PC) in Cyclohexan (37° C). 100 mcl einer SA-PC-Lösung (10 mg/ml $CHCl_3$) wurden verblasen (Argon) und der Rückstand in 1 ml Cyclohexan aufgenommen. Nach Hinzufügen einer solchen Menge Antioxidans, die sich auch in Olivenöl lösen würde (vgl. Tabelle 1), wurde die Extinktion bei 234 nm (Spektralphotometer Perkin Elmer 5528, Überlingen, FRG) gemessen und anschließend das Lösungsmittel mit Luft verblasen. Nach weiteren 24 Stunden Stehen im offenen Gefäß wurde der Rückstand wieder in 1 ml Cyclohexan gelöst und die Extinktion bei 234 nm als Maß des oxidierten SA-PC gemessen.

**Ergebnis:**

Wegen ihrer guten Lipidlöslichkeit konnte die Oxidation des SA-PC mit jeweils 1 mg bis 10 mg/ml der erfindungsgemäßen Antioxidatien praktisch vollständig unterdrückt werden. Die Oxidation des SA-PC konnte mit den erfindungsgemäßen reduzierten Dithiolverbindungen (Beispiele 49 und 50) überraschenderweise ebenso wirkungsvoll verhindert werden wie mit den entsprechenden oxidierten Dithio-Verbindungen (Beispiele 51 und 52 ohne freie SH-Gruppen).

**Methode V**

Inhibierende Wirkung auf die Fettsäureoxidation in Rattenmitochondrien

(Malonaldehyd-Bestimmung nach der Thiobarbiturmethode gemäß Ottolenghi, Arch. Biochem. Biophys. 79 (1959 S. 355 ff.). Die Bestimmung erfolgte spektralphotometrisch in Mitochondrialhomogenaten der Ratte. Die Inhibierung der Fettsäureoxidation durch die erfindungsgemäßen Präparate war praktisch vollständig.

**Methode VI**

Inhibierung der Lipidoxidation in liposomalen Biomembranen

Eine wäßrige Lösung von Liposomen (Fa. Nattermann, Köln/Deutschland) wurde mit aqua bidest verdünnt, bis die im Spektralphotometer (PMQ II der Firma zeiss, Oberkochen/Deutschland) bei 234 nm gegen Luft gemessene Extinktion zwischen 0.25 und 0,35 lag (entspricht etwa 0.1 mg Liposomen/ml). Diese Liposomensuspension wurde sodann mit 50 mcmol/l Cumolhydroperoxid und 12 mcmol Hämatin versetzt (Gesamtvolumen 1 ml) und die Zunahme der Extinktion bei 234 nm als Maß für die Geschwindigkeit der Liposomen-Oxidation zeitlich verfolgt.

Durch Zusatz der erfindungsgemäßen Antioxidantien konnte die Oxidation der Liposomen unterdrückt werden. Die folgende Tabelle zeigt die durchschnittliche Zunahme von $\Delta E_{234}$/min in Ansätzen mit und ohne Antioxidans nach 25 min bei 25° C.

**Tabelle:**

| | $\Delta E_{234}$/min |
|---|---|
| ohne Zusatz von Antioxidans | 0,035 |
| mit Zusatz von 100 nmol/l Antioxidantien | 0,005 bis 0,015 |

Noch bessere Ergebnisse werden erzielt, wenn die lipophilen Antioxidantien bei der Herstellung der Liposomen als Bestandteile derselben mit eingebaut werden.

**Methode VII**

Inhibierung der LDL-Oxidation nach El-Saadani et al., Journal of Lipid Research 30 (1989) Seite 627

Analog zu Methode VI schützen die lipophilen Antioxidantien auch "Low Density Lipoprotein" (Fa. Sigma, St. Louis, USA) vor Oxidation.

**Methode VIII**

Laser-induzierte Thrombose in Ratten in vivo.

Die experimentelle Durchführung erfolgte in allen Einzelheiten wie in der US-Patentschrift 4 694 024 beschrieben. Die Laser-Induktion erfolgte 60 min nach oraler Applikation der Antioxidantien. Nach oraler Gabe der erfindungsgemäßen Antioxidatien (30 mg/kg) war eine signifikante höhere Anzahl von Laserschüssen erforderlich als in den Vergleichsexperimenten, d.h. die thrombosehemmende Widerstandskraft der Tiere war nach Gabe der erfindungsgemäßen Antioxidantien höher.

| | Reduktion der Thrombusbildung |
|---|---|
| Verbindung gem. Beispiel 13 | 17 % |
| " " " 52 | 14 % |
| " " " 49 | 20 % |
| " " " 47 | 15 % |
| " " " 3 | 11 % |
| " " " 2 | 14 % |
| " " " 1 | 16 % |
| " " " 36 | 17 % |
| " " " 33 | 13 % |
| (4,4'-(Isopropylidene-dithio)bis-[2,6-di-tert.-butylphenol] | 6 % |
| Vitamin E | 5 % |

**Methode IX**

Photochemisch induzierte Thrombusbildung in Ratten in vivo.
Die Messungen wurden an Mesenterialarteriolen durchgeführt. Dazu wurden 0,3 ml einer Lösung von

Fluorescein-Isothiocyanat-Dextran-70 (FITC-Dextran, Fa. Sigma , Seidenhofen, FRG) injiziert, und daraufhin die Arteriolen im Beobachtungsfeld mit Licht (490 nm) bestrahlt. Die sich daraufhin bildenden Thromben wurden vitalmikroskopisch quantifiziert, wie unter Methode VIII beschrieben. Mit erfindungsgemäßen Antioxidantien konnte die Thrombusbildung eine Stunde nach oraler Gabe von 50 mg/kg Körpergewicht Ratte bis zu 20 % inhibiert werden.

**Methode X**

Arachidonsäure-induzierte Thrombozytenaggregation nach Ruppert und Weithmann, Life Sciences 31 (1982) 2037 f.

Die antithrombotische Wirkung der erfindungsgemäßen Substanzen kommt nicht durch eine Hemmung der Trombozytenaggregation zustande, denn bis 10 mcmol/l der erfindungsgemäßen Substanzen war keine signifikante Hemmwirkung nachzuweisen. Eine erhöhte Blutungsneigung von Patienten, die mit diesen Substanzen behandelt werden, ist also nicht zu erwarten.

Methode XI

Wirkung der erfindungsgemäßen Verbindungen bei Langzeitgabe an der hyperlipidämischen infarktempfindlichen Ratte:

Männliche infarktempfindliche, etwa 200 g schwere Tiere (Möllegaard, Ejby, Dänemark) wurden einmal täglich per os mit 1 ml/100 g Körpergewicht einer Standarddiät (100 g Cholsäure, 100 g Cholesterin, 30 g Propylthiouracil ad 1 l Sesamöl) behandelt. Während der Kontrollgruppe I (vgl. nachfolgende Tabelle) keine Prüfsubstanz verabfolgt wurde, enthielt die Standarddiät in den Verumexperimenten II - IV zusätzlich 50 mg/kg Körpergewicht der in der nachstehenden Tabelle angegebenen Prüfsubstanzen. Nach 9 Tagen wurden die Ratten, wie oben beschrieben, in der Laser induzierten Thrombose untersucht, sowie der Gesamtcholesteringehalt des Serums bestimmt. Aus der nachfolgenden Tabelle geht hervor, daß die Thromboseneigung der im Vergleich zu gesunden Ratten (<100 mg Cholesterin/dl) hyperlipidämischen infarktempfindlichen Ratten mit den erfindungsgemäßen Substanzen überraschenderweise erfolgreicher behandelt werden können als mit Vitamin E. Darüber hinaus übten die erfindungsgemäßen Substanzen eine vorteilhafte lipidsenkende Wirkung aus.

| | Gruppe | Zahl der Tiere n = | Gesamtcholesterin mg/dl | Reduktion der Thrombose-Neigung vs. Kontrolle (%) |
|---|---|---|---|---|
| I | Kontrolle | 5 | 286 | -- |
| II | Vitamin E | 6 | 278 | 26 |

| | Gruppe | Zahl der Tiere n = | Gesamtcholesterin mg/dl | Reduktion der Thrombose-Neigung vs. Kontrolle (%) |
|---|---|---|---|---|
| III | Verbindung gemäß Beispiel 2 | 4 | 270 | 50 |
| IV | Verbindung gemäß Beispiel 49 | 5 | 259 | 34 |

## Patentansprüche

1. Verbindungen der allgemeinen Formel I

   $(A)_a(L)(X)_{a'}$ (I),

   worin a, a', A, L und X die folgende Bedeutung besitzen:
   a und a' = unabhängig voneinander die Zahlen 1 oder 2,
   A = antioxidative Komponente aus der Gruppe
   $A_1$ - chromanteilstruktur des Vitamins E

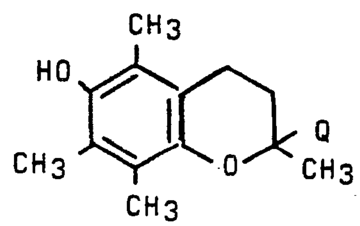

   worin Q in dieser und allen folgenden Formeln eine freie kovalente Einfachbindungs-Valenz darstellt,
   $A_2$ - alkylsubstituierter Mono-, Di- oder Tri-Phenol-Rest

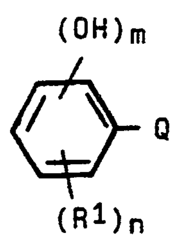

   worin
   m = 1 oder 2,
   n = 1 oder 2 und
   m+n = 3 oder 4,
   $R^1$ = Alkylrest und/oder Alkoxyrest
   und die Gesamtzahl der C-Atome des Alkyl - bzw. Alkoxyrestes bzw. der Alkyl- und Alkoxyreste = maximal 8;
   $A_3$ - Reductonrest

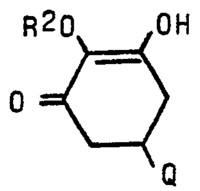

oder

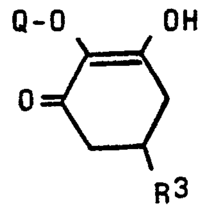

worin

$R^2$ = H oder niederer Alkylrest (vorzugsweise $C_1-C_4$) und

$R^3$ = H, $COOR^4$, $CH_2OR^4$

$R^4$ = H oder niederer Alkylrest (vorzugsweise $C_1-C_4$)

$A_4$ - 1,2-Dithiacycloalkyl- oder 1,2-Dithiacycloalkenyl-Rest mit 2 - 6, vorzugsweise 2 - 4 C-Atomen im Ring und die durch Hydrogenierung reduzierte Dithiolform dieser Reste

$A_5$ - Ascorbinsäure(-Derivat)-Rest

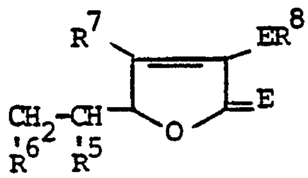

worin

E = O, S oder $NR^9$

$R^5$ = H, EH, EQ oder Q

$R^6$ = H, EH, EQ-(L-$X_1$) oder Q-(L-$X_1$)

$R^7$ = H, EH, EQ, Q oder einer der unter $A_2$ und $A_3$ genannten Reste,

$R^8$ = H, EH, Q-(L-$X_1$) oder -PO($OR^9$ )$_2$,

$R^9$ = H, niederer Alkylrest (vorzugsweise $C_1-C_4$) oder Q,

und nur 1 oder 2 - bevorzugt 1 - der Reste $R^5$ -$R^9$ gleich Q sind bzw. Q enthalten,

L = Brückenglied und

$X_1$ = lipophile Komponeten wie nachstehend definiert;

L = Brückenglied,

bestehend aus einem oder mehreren der Bausteine

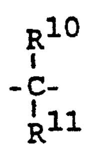 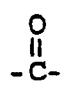 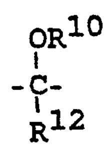 .

-O- , -S- , $-NR^{10}-$ und 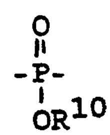

worin

$R^{10}$, $R^{11}$, $R^{12}$ = H, niederer Alkylrest (vorzugsweise $C_1-C_4$) oder Q,

$R^{11}$ darüber hinaus auch noch $-CO_aR^{10}$ sein kann (mit a = 1 oder 2),

und 2 Reste der Art -O-, -S- und/oder $-NR^{10}-$ durch mindestens 1 C- oder P-Atom voneinander getrennt sind;

X = lipophile Komponente aus der Gruppe

$X_1$ - Cholanderivat-Reste

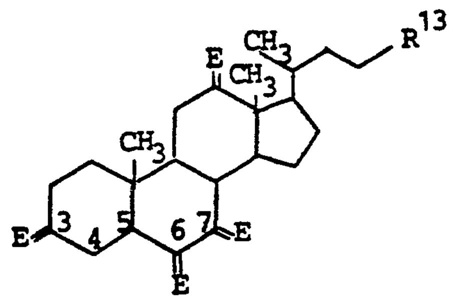

worin

$R^{13}$ = sec. $C_4H_9$ (= Cholestan), $R^{11}$ (s. bei L) oder Q,

E = O, S, $NR^{10}$ ($R^{10}$ s. bei L), (α,β-OH,H) oder (α,β-Q, H) und in 4,5- bzw. 5,6- bzw. 7,8-Position eine Doppelbindung vorhanden sein kann, und

$X_2$ - Alkyl- oder Cycloalkylrest oder Fettsäurederivat-Rest mit bis zu 24 C-Atomen.

**2.** Verbindungen der Formel I gemäß Anspruch 1, dadurch gekennzeichnet, daß die Komponente $A_4$ ein Rest der folgenden Formeln in der Dithiaform (gemäß den Formeln) oder in der durch Hydrogenierung reduzierten Dithiolform ist:

$A_{4.1}$

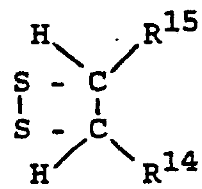

$A_{4.2}$

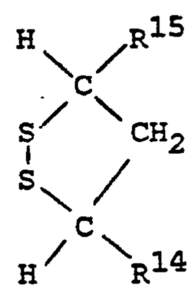

worin

$R^{14}$ = H oder niederer Alkylrest (vorzugsweise $C_1$-$C_4$), und

$R^{15}$ = $-(CH_2)_b-Q$

b = 0 - 12, vorzugsweise 0 - 4,

$A_{4.3}$

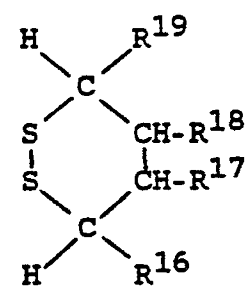

worin

| | |
|---|---|
| $R^{16}$ und $R^{19}$ = | unabhängig voneinander = H oder niederer Alkylrest (vorzugsweise $C_1$-$C_4$) |
| $R^{17}$ = | Q und |
| $R^{18}$ = | H, niederer Alkylrest (vorzugsweise $C_1$-$C_4$),Acylrest $OCOR^{19}$ oder $OR^{19}$ |
| $R^{19}$ = | niederer Alkylrest (vorzugsweise $C_1$-$C_4$) oder Q. |

$A_{4.4}$ Dithiothreit- oder Dithioerythrit-Teilstruktur

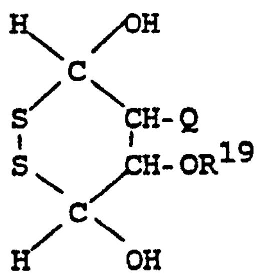

worin
$R^{19}$ die gleiche Bedeutung wie bei 4.3 besitzt,

$A_{4.5}$

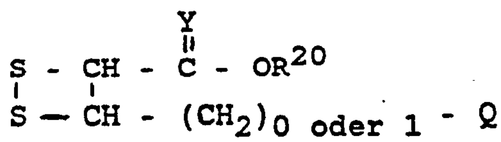

worin

| | |
|---|---|
| $R^{20}$ = | H oder niederer Alkylrest (vorzugsweise $C_1$-$C_4$) und |
| Y = | $H_2$ oder O. |

3. Verbindungen der Formel I gemäß Anspruch 2, dadurch gekennzeichnet, daß die Komponente

$A_{4.2}$
$R^{14}$ = H und
$R^{15}$ = $-(CH_2)_4$-Q
(= Decarboxy-Liponsäure- bzw. Dihydroliponsäure-Teilstruktur).

4. Verbindungen der Formel I gemäß Anspruch 1, dadurch gekennzeichnet, daß in dem Strukturrest

$A_5$ $R^7$ =

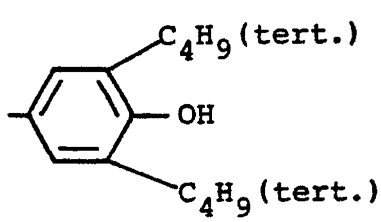

5. Verbindungen der Formel I gemäß Anspruch 1, dadurch gekennzeichnet, daß im Strukturrest
$A_5$
E = O
$R^5$, $R^6$ und $R^7$ = unabhängig voneinander = OH oder OQ,
$R^8$ = H oder Q,
wobei nur 1 oder 2 der Reste $R^5$ - $R^8$ Q enthalten bzw. gleich Q ist (=Ascorbinsäurerest).

6. Verbindungen der Formel I gemäß einem oder mehreren der Ansprüche 1 - 5, dadurch gekennzeichnet, daß das Brückenglied L die folgende allgemeine Formel besitzt:

$L = M_p\{[-(CH_2)_w-(G_1)_x-(G_2)]_v-(CH_2)_y-(G_3)_z-(G_4)_{p+1}\}M_p$

worin
p, x und z unabhängig voneinander = 0 oder 1,
v, w und y unabhängig voneinander = 0 - 4, und
v+w+y+z = 0 - 10,

M = $-CR^{10}=$ ,
$-N=$ oder
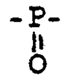

$G_1$, $G_2$, $G_3$ und $G_4$ unabhängig voneinander = -O- , -S- , $-NR^{10}-$ ,

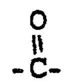 , $-CHOR^{10}-$ oder $-CH(CH_2-OR^{10})-$ ,

wobei $R^{10}$ die vorher genannte Bedeutung besitzt (= H, niederer Alkylrest oder Q) und 2 der Reste -O-, -S-, und/oder $-NR^{10}-$ durch mindestens 1 C-Atom voneinander getrennt sind.

7. Verbindungen der Formel I nach einem oder mehreren der Ansprüche 1 - 6, dadurch gekennzeichnet, daß das Brückenglied L ein Rest ist aus der Gruppe:
$L_1$ : $Q-O-(CH_2)_r-O-CO-Q$
$L_2$ : $Q-CO-NH-(CH_2)_q-NH-CO-Q$
$L_3$ : $Q-O-(CH_2-)_r-NH-CO-Q$
$L_4$ : $Q-(CH_2-)_r(-O-)_b-Q$
$L_5$ : $Q-(CH_2-)_s-O-(CH_2-)_r-O-Q$
$L_6$ : $Q-(CH_2-)_s-NH-(CH_2-)_r-O-Q$
$L_7$ : $Q-CO-NH-(CH_2-CH_2)_r-O-Q$
$L_8$ : $Q-O-(CH_2-)_s-CHOH-(CH_2-)_s-O-(CH_2-)_s-Q$

$L_9$ : 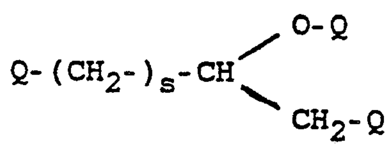

$L_{10}$: $Q-(CH_2-)_q-Q$
$L_{11}$: $Q-(CH_2-)_s-CHCO_2R^{10}-CHOH-Q$
$L_{12}$: $Q-CH=C(CO_2R^{10})-CO-Q$
$L_{13}$: $Q-CO-NH-(CH_2-)q-NH-CO-Q$
$L_{14}$: $Q-(CH_2-)_s-O-(CH_2-)_r-O-$

$L_{15}$: 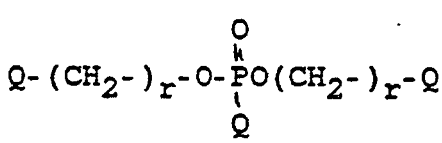

$L_{16}$: $[Q-(CH_2-)_2-O-(CH_2)_s]_2CH-Q$

$L_{17}$: Q-O-$(CH_2-)_s$-CHOH-O-$(CH_2-)_s$-Q
$L_{18}$: Q-O-$(CH_2-)_s$-CH($CH_2$-OH)-O-CO-Q
$L_{19}$: Q-O-$(CH_2-)_s$-CHOH-$(CH_2-)_s$-O-CO-O
$L_{20}$: Q-CO-$NR^{10}$-Q
$L_{21}$: Q-$CO(O)_x$-Q
$L_{22}$: Q-$CH_2$-N[$CH(CH_3)_2$]-$(CH_2)_r$-CHOHCH$_2$CHOHCH$_2$-$CO(O)_x$-Q
$L_{23}$: Q-$(CH_2)_s$-Q
$L_{24}$: Q-$NR^{10}$-Q
$L_{25}$: Q-O-Q
$L_{26}$: Q-$(CH_2)_s$-CHCO$_2$R$^{10}$-SOQ

$L_{27}$: 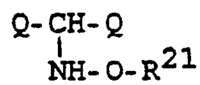

worin $R^{21}$ = Benzyl- oder $R^{10}$

$L_{28}$: 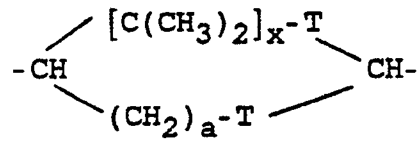

worin
T = O oder S
x = 0, 1
a = 1, 2 und
q = 1 - 5, bevorzugt 3
r = 1 - 5, bevorzugt 2
s = 1 - 5, bevorzugt 1 bedeuten,
und $R^{10}$ die oben genannte Bedeutung besitzt
(= H, niederer Alyklrest oder Q).

**8.** Verbindungen der Formel I nach einem oder mehreren der Ansprüche 1 - 7, dadurch gekennzeichnet, daß die lipophile Komponente X ein Rest aus der folgenden Gruppe ist:
$X_{1.1}$ Cholesterol
$X_{1.2}$ Cholestanol
$X_{1.3}$ Cholsäure
$X_{1.4}$ Desoxycholsäure
$X_{1.5}$ Ursodesoxycholsäure
$X_{1.6}$ Chenodesoxycholsäure.

**9.** Verbindungen der Formel I nach einem oder mehreren der Ansprüche 1 - 7, dadurch gekennzeichnet, daß die lipophile Komponente X ein Rest aus der folgenden Gruppe ist:
$X_{2.1}$ $CH_3$-$(CH_2)_t$-Q
$X_{2.2}$ Q-$C(CH_3)_3$
$X_{2.3}$ Q-$CH(CH_2)_d$
$X_{2.4}$ Q-C≡C-$(CH_2)_5$-$CH_3$
$X_{2.5}$ $R^{10}$-$CO_2$-$(CH_2)_z$-Q
d = 4 - 6
t = 3 - 24, vorz. 6 - 18
z = 0 oder 1.

**10.** Verfahren zur Herstellung einer Verbindung der Formel I gemäß der Definition in einem oder mehreren der Ansprüche 1 - 9, dadurch gekennzeichnet, daß man die Einzelkomponenten A und X in freier oder geschützter Form oder auch in Form reaktiver Derivate mit einem reaktiven Derivat von L umsetzt und anschließend gegebenenfalls vorhandene Schutzgruppen abspaltet.

**11.** Verwendung der Verbindungen der Formel I gemäß der Definition in einem oder mehreren der

Ansprüche 1 - 9 als Antioxidantien.

**12.** Ausführungsform gemäß Anspruch 11, dadurch gekennzeichnet, daß die Verbindungen als Antioxidantien in der Lebensmittel- und Kosmetikindustrie verwendet werden.

**13.** Verwendung der Verbindungen der Formel I gemäß der Definition in einem oder mehreren der Ansprüche 1 - 9 als Heilmittel, vorzugsweise für Krankheiten, bei denen Bioradikale involviert sind.

**14.** Ausführungsform gemäß Anspruch 13, dadurch gekennzeichnet, daß die Verbindungen als Heilmittel für Herz-Kreislauf- und Gefäßkrankheiten verwendet werden.

**15.** Arzneimittel, gekennzeichnet durch einen Gehalt an einer wirksamen Menge einer Verbindung der Formel I gemäß der Definition in einem oder mehreren der Ansprüche 1 - 9, neben üblichen pharmakologisch verträglichen Träger- und/oder Zusatzstoffen.

**Patentansprüche für folgende Vertragsstaaten: ES, GR,**

**1.** Verfahren zur Herstellung einer Verbindungen der allgemeinen Formel I

$(A)_a(L)(X)_{a'}$ (I),

worin a, a', A, L und X die folgende Bedeutung besitzen:
a und a' = unabhängig voneinander die Zahlen 1 oder 2,
A = antioxidative Komponente aus der Gruppe
$A_1$ - chromanteilstruktur des Vitamins E

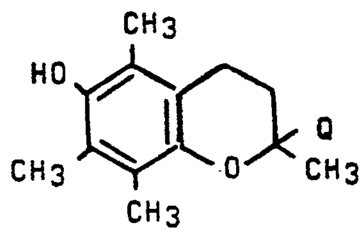

worin Q in dieser und allen folgenden Formeln eine freie kovalente Einfachbindungs-Valenz darstellt,
$A_2$ - alkylsubstituierter Mono-, Di- oder Tri-Phenol-Rest

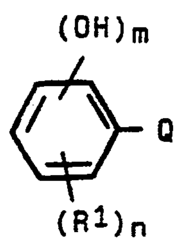

worin
m = 1 oder 2,
n = 1 oder 2 und
m + n = 3 oder 4,
$R^1$ = Alkylrest und/oder Alkoxyrest
und die Gesamtzahl der C-Atome des Alkyl- bzw. Alkoxyrestes bzw. der Alkyl- und Alkoxyreste = maximal 8;
$A_3$ - Reductonrest

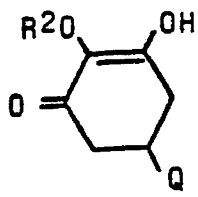

oder

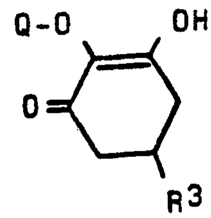

worin

$R^2$ = H oder niederer Alkylrest (vorzugsweise $C_1$-$C_4$) und

$R^3$ = H, $COOR^4$, $CH_2OR^4$

$R^4$ = H oder niederer Alkylrest (vorzugsweise $C_1$-$C_4$)

$A_4$ - 1,2-Dithiacycloalkyl- oder 1,2-Dithiacycloalkenyl-Rest mit 2 - 6, vorzugsweise 2 - 4 C-Atomen im Ring und die durch Hydrogenierung reduzierte Dithiolform dieser Reste

$A_5$ - Ascorbinsäure(-Derivat)-Rest

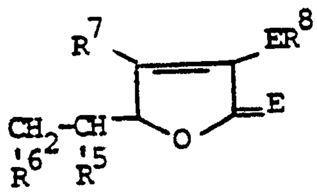

worin

E = O, S oder $NR^9$

$R^5$ = H, EH, EQ oder Q

$R^6$ = H, EH, EQ-(L-$X_1$) oder Q-(L-$X_1$)

$R^7$ = H, EH, EQ, Q oder einer der unter $A_2$ und $A_3$ genannten Reste,

$R^8$ = H, EH, Q-(L-$X_1$) oder -$PO(OR^9)_2$,

$R^9$ = H, niederer Alkylrest (vorzugsweise $C_1$-$C_4$) oder Q,

und nur 1 oder 2 - bevorzugt 1 - der Reste $R^5$ -$R^9$ gleich Q sind bzw. Q enthalten,

L = Brückenglied und

$X_1$ = lipophile Komponeten wie nachstehend definiert;

L = Brückenglied,

bestehend aus einem oder mehreren der Bausteine

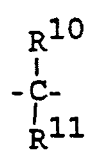

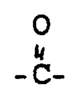

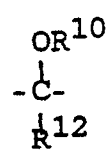

-O- , -S- , -$NR^{10}$- und 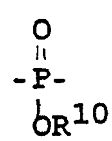

worin

$R^{10}$, $R^{11}$, $R^{12}$ = H, niederer Alkylrest (vorzugsweise $C_1$-$C_4$) oder Q,

$R^{11}$ darüber hinaus auch noch -$CO_aR^{10}$ sein kann (mit a = 1 oder 2),

und 2 Reste der Art -O-, -S- und/oder -$NR^{10}$- durch mindestens 1 C- oder P-Atom voneinander getrennt sind;

X = lipophile Komponente aus der Gruppe

$X_1$ - Cholanderivat-Reste

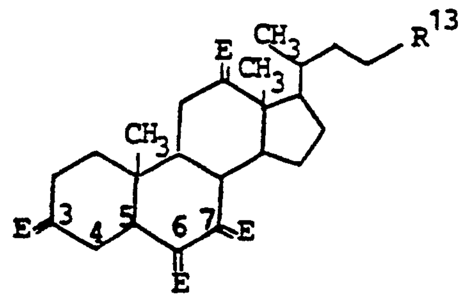

worin

$R^{13}$ = sec. $C_4H_9$ (= Cholestan), $R^{11}$ (s. bei L) oder Q,

E = O, S, $NR^{10}$ ($R^{10}$ s. bei L), ($\alpha,\beta$-OH H) oder ($\alpha,\beta$-Q, H) und in 4,5- bzw. 5,6- bzw. 7,8-Position eine Doppelbindung vorhanden sein kann, und

$X_2$ - Alkyl- oder Cycloalkylrest oder Fettsäurederivat-Rest mit bis zu 24 C-Atomen,

dadurch gekennzeichnet, daß man die Einzelkomponenten A und X in freier oder geschützter Form oder auch in Form reaktiver Derivate mit einem reaktiven Derivat von L umsetzt und anschließend gegebenenfalls vorhandene Schutzgruppen abspaltet.

2. Verfahren gemäß Anspruch 1, dadurch gekennzeichnet, daß die Komponente $A_4$ ein Rest der folgenden Formeln in der Dithiaform (gemäß den Formeln) oder in der durch Hydrogenierung reduzierten Dithiolform ist:

$A_{4.1}$

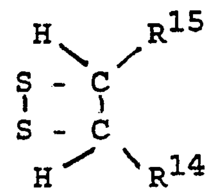

$A_{4.2}$

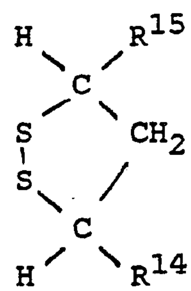

worin

$R^{14}$ = H oder niederer Alkylrest (vorzugsweise $C_1$-$C_4$), und

$R^{15}$ = $(CH_2)_b$-Q

b = 0 - 12, vorzugsweise 0 - 4,

$A_{4.3}$

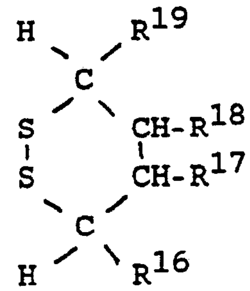

worin

| | |
|---|---|
| $R^{16}$ und $R^{19}$ = | unabhängig voneinander = H oder niederer Alkylrest (vorzugsweise $C_1$–$C_4$) |
| $R^{17}$ = | Q und |
| $R^{18}$ = | H, niederer Alkylrest (vorzugsweise $C_1$–$C_4$),Acylrest $OCOR^{19}$ oder $OR^{19}$ |
| $R^{19}$ = | niederer Alkylrest (vorzugsweise $C_1$–$C_4$) oder Q, |

$A_{4.4}$ Dithiothreit- oder Dithioerythrit-Teilstruktur

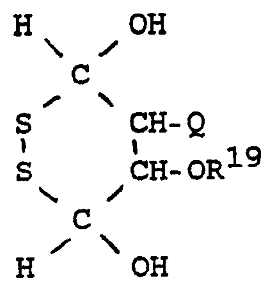

worin
$R^{19}$ die gleiche Bedeutung wie bei 4.3 besitzt,

$A_{4.5}$

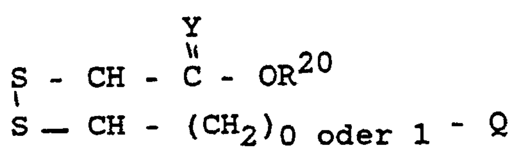

worin

| | |
|---|---|
| $R^{20}$ = | H oder niederer Alkylrest (vorzugsweise $C_1$–$C_4$) und |
| Y = | $H_2$ oder O ist, hergestellt wird. |

**3.** Verfahren gemäß Anspruch 2, dadurch gekennzeichnet, daß die Komponente

$A_{4.2}$
$R^{14}$ = H und
$R^{15}$ = $-(CH_2)_4-Q$
(= Decarboxy-Liponsäure- bzw. Dihydroliponsäure-Teilstruktur) hergestellt wird.

**4.** Verfahren gemäß Anspruch 1, dadurch gekennzeichnet, daß man den Strukturrest $A_5$, wobei $R^7$ für

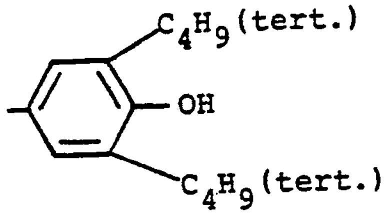

steht, herstellt.

5. Verfahren gemäß Anspruch 1, dadurch gekennzeichnet, daß man den Strukturrest $A_5$, wobei E = O
$R^5$, $R^6$ und $R^7$ = unabhängig voneinander = OH oder OQ,
$R^8$ = H oder Q,
wobei nur 1 oder 2 der Reste $R^5$ - $R^8$ Q enthalten bzw. gleich Q ist (=Ascorbinsäurerest), herstellt.

6. Verfahren gemäß einem oder mehreren der Ansprüche 1 bis 5, dadurch gekennzeichnet, daß man das Brückenglied L mit folgender allgemeiner Formel:

$L = M_p\{[-(CH_2)_w-(G_1)_x-(G_2)]_v-(CH_2)_y-(G_3)_z-(G_4)_{p+1}\}M_p$

worin
p, x und z unabhängig voneinander = 0 oder 1,
v, u und y unabhängig voneinander = 0 - 4, und
v+w+y+z = 0 - 10,

$M = -CR^{10}=$ ,
-N= oder
-P-
‖
O

$G_1$, $G_2$, $G_3$ und $G_4$ unabhängig voneinander = -O- , -S- , $-NR^{10}-$ ,

O
‖
-C- , $-CHOR^{10}-$ oder $-CH(CH_2-OR^{10})-$ ,

wobei $R^{10}$ die vorher genannte Bedeutung besitzt (= H, niederer Alkylrest oder Q) und 2 der Reste -O-, -S-, und/oder $-NR^{10}-$ durch mindestens 1 C-Atom voneinander getrennt sind, herstellt.

7. Verfahren nach einem oder mehreren der Ansprüche 1 bis dadurch gekennzeichnet, daß man das Brückenglied L mit einem Rest aus der Gruppe:
$L_1$ : $Q-O-(CH_2)_r-O-CO-Q$
$L_2$ : $Q-CO-NH-(CH_2)_q-NH-CO-Q$
$L_3$ : $Q-O-(CH_2-)_r-NH-CO-Q$
$L_4$ : $Q-(CH_2-)_r(-O-)_b-Q$
$L_5$ : $Q-(CH_2-)_s-O-(CH_2-)_r-O-Q$
$L_6$ : $Q-(CH_2-)_s-NH-(CH_2-)_r-O-Q$
$L_7$ : $Q-CO-NH-(CH_2-CH_2)_r-O-Q$
$L_8$ : $Q-O-(CH_2-)_s-CHOH-(CH_2-)_s-O-(CH_2-)_s-Q$

$L_9$ : 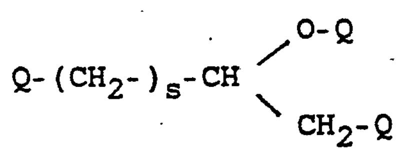

$L_{10}$: Q-$(CH_2-)_q$-Q
$L_{11}$: Q-$(CH_2-)_s$-$CHCO_2R^{10}$-CHOH-Q
$L_{12}$: Q-CH = C($CO_2R^{10}$)-CO-Q
$L_{13}$: Q-CO-NH-$(CH_2-)_q$-NH-CO-Q
$L_{14}$: Q-$(CH_2-)_s$-O-$(CH_2-)_r$-O-

$L_{15}$: 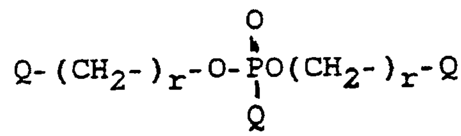

$L_{16}$: [Q-$(CH_2-)_2$-O-$(CH_2)_s]_2$CH-Q
$L_{17}$: Q-O-$(CH_2-)_s$-CHOH-O-$(CH_2-)_s$-Q
$L_{18}$: Q-O-$(CH_2-)_s$-CH($CH_2$-OH)-O-CO-Q
$L_{19}$: Q-O-$(CH_2-)_s$-CHOH-$(CH_2-)_s$-O-CO-O
$L_{20}$: Q-CO-$NR^{10}$-Q
$L_{21}$: Q-CO$(O)_x$-Q
$L_{22}$: Q-$CH_2$-N[CH$(CH_3)_2$]-$(CH_2)_r$-$CHOHCH_2CHOHCH_2$-CO$(O)_x$-Q
$L_{23}$: Q-$(CH_2)_s$-Q
$L_{24}$: Q-($NR^{10}$-Q
$L_{25}$: Q-O-Q
$L_{26}$: Q-$(CH_2)_s$-$CHCO_2R^{10}$-SOQ
$L_{27}$: Q-CH-Q
NH-O-$R^{21}$
worin
$R^{21}$ = Benzyl- oder $R^{10}$

$L_{28}$: 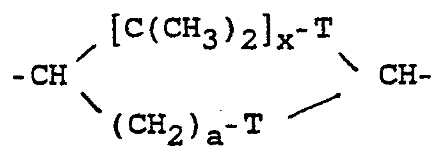

worin
T = O oder S
x = 0, 1
a = 1, 2 und
q = 1 - 5, bevorzugt 3
r = 1 - 5, bevorzugt 2
s = 1 - 5, bevorzugt 1 bedeuten,
und $R^{10}$ die oben genannte Bedeutung besitzt ( = H, niederer Alyklrest oder Q), herstellt.

**8.** Verfahren nach einem oder mehreren der Ansprüche 1 bis 7, dadurch gekennzeichnet, daß man die lipophile Komponente X mit einem Rest aus der folgenden Gruppe:
$X_{1.1}$ Cholesterol
$X_{1.2}$ Cholestanol
$X_{1.3}$ Cholsäure
$X_{1.4}$ Desoxycholsäure
$X_{1.5}$ Ursodesoxycholsäure
$X_{1.6}$ Chenodesoxycholsäure, herstellt.

**9.** Verfahren nach einem oder mehreren der Ansprüche 1 bis 7, dadurch gekennzeichnet, daß man die lipophile Komponente X mit einem Rest aus der folgenden Gruppe:
$X_{2.1}$ $CH_3$-$(CH_2)_t$-Q
$X_{2.2}$ Q-C$(CH_3)_3$
$X_{2.3}$ Q-CH$(CH_2)_d$

$X_{2.4}$ Q-C≡C-$(CH_2)_5$-$CH_3$
$X_{2.5}$ $R^{10}$-$CO_2$-$(CH_2)_z$-Q
d = 4 - 6
t = 3 - 24, vorg. 6 - 18
z = 0 oder 1, herstellt.

**10.** Verwendung der Verbindungen der Formel I gemäß der Definition in einem oder mehreren der Ansprüche 1 - 9 als Antioxidantien.

**11.** Verwendung der Verbindung der Formel I gemäß Anspruch 10, als Antioxidantien in der Lebensmittel- und Kosmetikindustrie.

**12.** Verwendung der Verbindungen der Formel I gemäß der Defintion in einem oder mehreren der Ansprüche 1 - 9 zur Herstellung eines Arzneimittels zur Behandlung von Krankheiten, bei denen Bioradikale involviert sind.

**13.** Verwendung der Verbindungen der Formel I, gemäß Anspruch 12 zur Herstellung eines Arzneimittels zur Behandlung von Herz-Kreislauf- und Gefäßkrankheiten.

**14.** Verfahren zur Herstellung eines Arzneimittels, dadurch gekennzeichnet, daß man mindestens eine Verbindung der Formel I gemäß der Definition in einem oder mehreren der Ansprüche 1 - 9, neben üblichen pharmakologisch verträglichen Träger- und/oder Zusatzstoffen in eine geeignete Darreichungsform bringt.